(19) Europäisches Patentamt
European Patent Office
Office européen des brevets

(11) EP 4 628 103 A1

(12) EUROPEAN PATENT APPLICATION
published in accordance with Art. 153(4) EPC

(43) Date of publication:
**08.10.2025 Bulletin 2025/41**

(21) Application number: **23896775.6**

(22) Date of filing: **28.11.2023**

(51) International Patent Classification (IPC):
***A61K 47/68*** *(2017.01)* ***A61K 39/395*** *(2006.01)*
***A61P 35/00*** *(2006.01)*

(52) Cooperative Patent Classification (CPC):
**A61K 39/395; A61K 47/68; A61P 35/00**

(86) International application number:
**PCT/CN2023/134686**

(87) International publication number:
**WO 2024/114626 (06.06.2024 Gazette 2024/23)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.11.2022 CN 202211512852**

(71) Applicants:
- **Suzhou Suncadia Biopharmaceuticals Co., Ltd.**
  **Suzhou, Jiangsu 215126 (CN)**
- **Shanghai Hengrui Pharmaceutical Co., Ltd.**
  **Shanghai 200245 (CN)**
- **Jiangsu Hengrui Pharmaceuticals Co., Ltd.**
  **Lianyungang, Jiangsu 222047 (CN)**

(72) Inventors:
- **YANG, Xiqin**
  **Suzhou, Jiangsu 215126 (CN)**
- **GE, Lingxiao**
  **Suzhou, Jiangsu 215126 (CN)**
- **YANG, Zhen**
  **Suzhou, Jiangsu 215126 (CN)**
- **WANG, Hongwei**
  **Suzhou, Jiangsu 215126 (CN)**

(74) Representative: **Potter Clarkson**
**Chapel Quarter**
**Mount Street**
**Nottingham NG1 6HQ (GB)**

Remarks:
The complete document including Reference Table(s) and the Sequence Listing(s) can be downloaded from the EPO website

# (54) ANTI-HER3 ANTIBODY-DRUG CONJUGATE COMPOSITION AND PHARMACEUTICAL USE THEREOF

(57) The present invention relates to an anti-HER3 antibody-drug conjugate composition and pharmaceutical use thereof. Specifically, the composition of the present invention comprises an anti-HER3 antibody-drug conjugate and a buffering agent.



Processed by Luminess, 75001 PARIS (FR)

## Description

### TECHNICAL FIELD

The present invention relates to a composition comprising an anti-HER3 antibody-drug conjugate (particularly an anti-HER3 antibody-exatecan analog conjugate) and pharmaceutical use thereof.

### BACKGROUND

The statements herein merely provide background information related to the present disclosure and may not necessarily constitute the prior art.

HER3 (epidermal growth factor receptor 3, ErbB-3, or HER3) is a member of the epidermal growth factor receptor (EGFR) family. This family includes HER1 (erbB1, EGFR), HER2 (erbB2, NEU), HER3 (erbB3), and HER4 (erbB4). Each of these receptors comprises 3 parts: an extracellular region, a transmembrane region, and an intracellular region. The extracellular region comprises 4 domains. The intracellular region comprises one intracellular tyrosine kinase domain for signaling and, in the cytoplasm, one tail with phosphorylatable tyrosine residues. When ligands bind to extracellular domains I and III, cell signaling is initiated. Normally, these receptors mediate cell division, migration, survival, and organ development. When the EGFR family members mutate, the resulting aberrant signaling stimulates cell survival, associated with cancer progression. The basic principle behind the activation and physiological action of the HER3 receptor is similar to those of the other family members, except that its ligands include neuregulin 1 (NRG-1) and neuregulin 2 (NRG-2) and that activated HER3 cannot form a homomer but only a heterodimer with EGFR or HER2. In the process of HER3 forming a heterodimer, its intracellular domain exhibits higher tyrosine phosphorylase activity. Structural analysis shows that the intracellular domain of HER3 has six P85 (PI-3K subunit) binding sites. This specific structure determines that HER3, when interacting with P85 regulatory subunits, can recruit up to six PI-3K to the regulatory subunit sites, thereby strongly activating the PI-3K signaling pathway. In fact, the HER3/HER2 dimer is the most active of the HER dimers. EGFRs are widely distributed on the surface of the cells such as mammalian epithelial cells, fibroblasts, glial cells, and keratinocytes. The EGFR signaling pathway plays an important role in physiological processes such as cell growth, proliferation, and differentiation.

HER3 is highly expressed in various common malignancies such as breast cancer, gastric cancer, ovarian cancer, prostate cancer, bladder cancer, colorectal cancer, head and neck squamous cell carcinoma, and melanoma. Unlike the EGFR gene, whose mutations result in its high-level expression or overactivation, the HER3 gene has a low mutation rate, and its high expression is mainly attributed to increased mRNA transcription, which leads to increased protein translation. In addition, HER3 is often highly expressed together with HER2, and the high expression of HER3 is closely associated with the development and progression of various tumors, as well as the survival of subjects. Therefore, the research on anti-tumor drugs targeting HER3 is of great significance.

ADCs have a more complex heterogeneous structure than antibodies, which presents greater challenges in developing ADC formulations for therapeutic purposes.

### SUMMARY

The present disclosure provides a pharmaceutical composition comprising an anti-HER3 antibody-drug conjugate, wherein the anti-HER3 antibody-drug conjugate has the structure shown below:

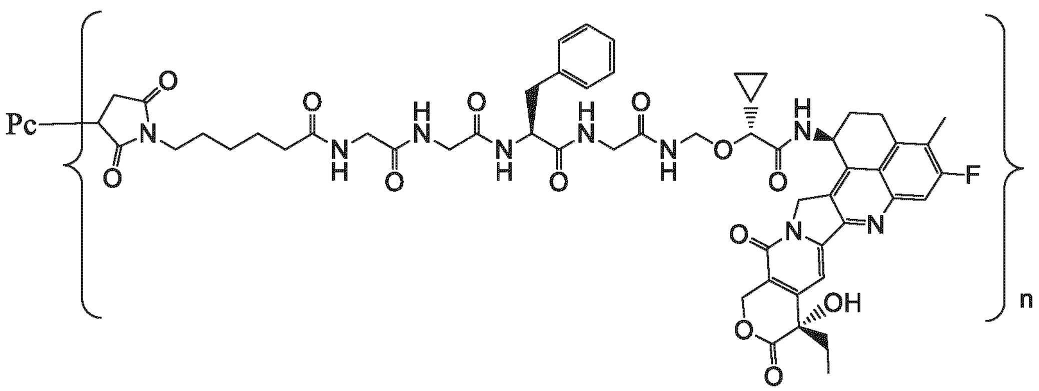

,

wherein:

n is 1 to 8, preferably 3 to 5;

Pc is an anti-HER3 antibody comprising a heavy chain variable region and a light chain variable region, wherein:

the heavy chain variable region comprises a HCDR1, a HCDR2, and a HCDR3 set forth in SEQ ID NO: 1, SEQ ID NO: 2, and SEQ ID NO: 3, respectively, and the light chain variable region comprises a LCDR1, a LCDR2, and a LCDR3 set forth in SEQ ID NO: 4, SEQ ID NO: 5, and SEQ ID NO: 6, respectively;

the pharmaceutical composition further comprises an acetate buffer, and the pharmaceutical composition has a pH of 4.9 to 5.5.

In an optional embodiment, the pharmaceutical composition has a pH of 5.0 to 5.5.

In an optional embodiment, the acetate buffer is an acetic acid-sodium acetate buffer.

In an optional embodiment, provided is the pharmaceutical composition according to any one of the foregoing, wherein n is 3 to 5, preferably about 4.0 (i.e., 4.0 ± 0.4), and more preferably 4.0.

In an optional embodiment, provided is the pharmaceutical composition according to any one of the foregoing, wherein the anti-HER3 antibody comprises a heavy chain variable region set forth in SEQ ID NO: 7 and a light chain variable region set forth in SEQ ID NO: 8.

In an optional embodiment, provided is the pharmaceutical composition according to any one of the foregoing, wherein the anti-HER3 antibody comprises a heavy chain set forth in SEQ ID NO: 9 and a light chain set forth in SEQ ID NO: 10.

In an optional embodiment, the pharmaceutical composition according to any one of the foregoing has a pH of 4.9 to 5.5, preferably a pH of 5.0 to 5.5; non-limiting examples include 4.9, 5.0, 5.1, 5.2, 5.3, 5.4, and 5.5, as well as any range between these point values; preferably, the pH is about 5.2; more preferably, the pH is 5.1 to 5.4; most preferably, the pH is 5.1 to 5.3. The "about" preceding the pH value indicates ±0.2; for example, about 5.0 means 5.0 ± 0.2 (that is, the pH is 4.8 to 5.2), and about 5.2 means 5.2 ± 0.2 (that is, the pH is 5.0 to 5.4).

In an optional embodiment, the pharmaceutical composition according to any one of the foregoing further comprises a surfactant. In some embodiments, the surfactant is selected from the group consisting of a polysorbate (e.g., polysorbate 20 or polysorbate 80), poloxamer, Triton, sodium dodecyl sulfonate, sodium lauryl sulfonate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauramidopropyl-betaine, cocamidopropyl-betaine, linoleamidopropyl-betaine, myristamidopropyl-betaine, palmitamidopropyl-betaine, isostearamidopropyl-betaine, myristamidopropyl-dimethylamine, palmitamidopropyl-dimethylamine, isostearamidopropyl-dimethylamine, sodium methyl cocoyl, sodium methyl oleyl taurate, polyethylene glycol, polypropylene glycol, copolymers of ethylene and propylene glycol, etc.Preferably, the surfactant is a polysorbate; more preferably, the surfactant is polysorbate 80 or polysorbate 20; most preferably, the surfactant is polysorbate 80.

In an optional embodiment, the surfactant in the pharmaceutical composition according to any one of the foregoing is at a concentration of 0.01 mg/mL to 1.0 mg/mL, preferably 0.05 mg/mL to 0.6 mg/mL, more preferably 0.1 mg/mL to 0.4 mg/mL, and most preferably about 0.2 mg/mL; non-limiting examples include 0.01 mg/mL, 0.02 mg/mL, 0.05 mg/mL, 0.1 mg/mL, 0.15 mg/mL, 0.2 mg/mL, 0.25 mg/mL, 0.3 mg/mL, 0.35 mg/mL, 0.4 mg/mL, 0.45 mg/mL, 0.5 mg/mL, 0.6 mg/mL, 0.7 mg/mL, 0.8 mg/mL, 0.9 mg/mL, and 1.0 mg/mL, as well as any range between these point values. The term "about" optimally indicates ±10%; for example, the concentration of polysorbate 80 is about 0.2 mg/mL, i.e., 0.2 mg/mL ± 0.02 mg/mL.

In some embodiments, the surfactant is at a concentration of about 0.4 mg/mL. In some embodiments, the surfactant is at a concentration of about 0.2 mg/mL. In some embodiments, the surfactant is at a concentration of 0.4 mg/mL. In some embodiments, the surfactant is at a concentration of 0.2 mg/mL.

In an optional embodiment, the pharmaceutical composition according to any one of the foregoing further comprises a sugar. As used herein, "sugar" includes the general composition $(CH_2O)_n$ and derivatives thereof, including monosaccharides, disaccharides, trisaccharides, polysaccharides, sugar alcohols, reducing sugars, non-reducing sugars, etc. The sugar may be selected from the group consisting of glucose, sucrose, trehalose, α,α-trehalose dihydrate, lactose, fructose, maltose, dextran, glycerin, erythritol, glycerol, arabitol, sylitol, sorbitol, mannitol, melibiose, melezitose, raffinose, mannotriose, stachyose, maltose, lactulose, maltulose, glucitol, maltitol, lactitol, iso-maltulose, etc. Preferably, the sugar is a non-reducing disaccharide; more preferably, the sugar is α,α-trehalose dihydrate or sucrose; most preferably, the sugar is sucrose.

In an optional embodiment, the sugar in the pharmaceutical composition according to any one of the foregoing is at a concentration of 10 mg/mL to 150 mg/mL, preferably 20 mg/mL to 120 mg/mL, more preferably 40 mg/mL to 120 mg/mL, and most preferably 40 mg/mL to 80 mg/mL. In some embodiments, the sugar is at a concentration of 48 mg/mL to 72 mg/mL. In some embodiments, the sugar is at a concentration of 54 mg/mL to 66 mg/mL. Non-limiting examples include 10 mg/mL, 25 mg/mL, 30 mg/mL, 35 mg/mL, 40 mg/mL, 45 mg/mL, 48 mg/mL, 50 mg/mL, 54 mg/mL, 55 mg/mL, 60 mg/mL, 66 mg/mL, 70 mg/mL, 72 mg/mL, 80 mg/mL, 90 mg/mL, 100 mg/mL, 120 mg/mL, and 150 mg/mL, as well as any range

between these point values; for example, preferably, the concentration is 60 mg/mL to 120 mg/mL; more preferably, the concentration is 60 mg/mL to 90 mg/mL; a point value is preferably about 60 mg/mL. The term "about" optimally indicates ±10%; for example, the concentration of sucrose is about 60 mg/mL, i.e., 60 mg/mL ± 6 mg/mL.

In an optional embodiment, the buffer in the pharmaceutical composition according to any one of the foregoing is at a concentration of 1 mM to 50 mM, preferably 10 mM to 20 mM. Non-limiting examples include 1 mM, 5 mM, 8 mM, 9 mM, 10 mM, 11 mM, 12 mM, 15 mM, 16 mM, 17 mM, 18 mM, 19 mM, 20 mM, 25 mM, 30 mM, 40 mM, and 50 mM, as well as any range between these point values. A non-limiting implementation range is 5 mM to 25 mM, preferably 5 mM to 15 mM, and more preferably about 10 mM. The term "about" optimally indicates ±10%; for example, the concentration of the acetic acid-sodium acetate buffer is about 10 mM, i.e., 10 mM ± 1 mM.

In an optional embodiment, the antibody-drug conjugate in the pharmaceutical composition according to any one of the foregoing is at a concentration of 1 mg/mL to 50 mg/mL, preferably 1 mg/mL to 40 mg/mL, and more preferably 20 mg/mL to 40 mg/mL, on a protein basis. Non-limiting examples include 1 mg/mL, 5 mg/mL, 10 mg/mL, 11 mg/mL, 12 mg/mL, 13 mg/mL, 14 mg/mL, 15 mg/mL, 16 mg/mL, 17 mg/mL, 18 mg/mL, 19 mg/mL, 20 mg/mL, 21 mg/mL, 22 mg/mL, 23 mg/mL, 24 mg/mL, 25 mg/mL, 26 mg/mL, 27 mg/mL, 28 mg/mL, 29 mg/mL, 30 mg/mL, and 40 mg/mL, as well as any range between these point values; preferably, the antibody-drug conjugate is at a concentration of 10 mg/mL to 30 mg/mL on a protein basis; more preferably, the antibody-drug conjugate is at a concentration of 15 mg/mL to 25 mg/mL on a protein basis; most preferably, the antibody-drug conjugate is at a concentration of about 20 mg/mL (18 mg/mL to 22 mg/mL) on a protein basis. Specifically, non-limiting examples include 20.1 mg/mL, 20.2 mg/mL, 20.3 mg/mL, 20.4 mg/mL, 20.5 mg/mL, 20.6 mg/mL, 20.7 mg/mL, 20.8 mg/mL, 20.81 mg/mL, 20.82 mg/mL, 20.83 mg/mL, 20.84 mg/mL, 20.85 mg/mL, 20.86 mg/mL, 20.87 mg/mL, 20.88 mg/mL, 20.89 mg/mL, 20.9 mg/mL, 20.91 mg/mL, 20.92 mg/mL, 20.93 mg/mL, 20.94 mg/mL, 20.95 mg/mL, 20.96 mg/mL, 20.97 mg/mL, 20.98 mg/mL, 20.99 mg/mL, and 21 mg/mL, as well as any range between these point values. The phrase "on a protein basis" means that the concentration is calculated based on the antibody moiety in the antibody-drug conjugate. The term "about" optimally indicates 10%; for example, the protein concentration is optimally about 20 mg/mL, i.e., 20 mg/mL ± 2 mg/mL.

In some embodiments, the anti-HER3 antibody is at a concentration of about 40 mg/mL. In some embodiments, the anti-HER3 antibody is at a concentration of about 20 mg/mL. In some embodiments, the anti-HER3 antibody is at a concentration of 40 mg/mL. In some embodiments, the anti-HER3 antibody is at a concentration of 20 mg/mL.

In an optional embodiment, the range of drug loading (n) may be the average number of cytotoxic drugs bound per anti-HER3 antibody; non-limiting examples include the average number of cytotoxic drugs bound per antibody being about 1, 2, 3, 4, 5, 6, 7, 8, 9, 10, 11, and 12, as well as any range between these point values. Preferably, the range of drug loading (n) is selected from the group consisting of 1-8, 2-8, 2-7, 2-6, 2-5, 2-4, 3-4, 3-5, 3.5-4.7, 3.6-4.4, 5-6, 5-7, 5-8, and 6-8. Illustratively, the drug loading (n) may be an average of 1, 2, 3, 4, 5, 6, 7, 8, 9, or 10. n is a decimal or integer. Non-limiting examples of n is about 4 (3.6-4.4).

In an optional embodiment, provided is the pharmaceutical composition according to any one of the foregoing, which comprises:

(a) on a protein basis, 1 mg/mL to 40 mg/mL said anti-HER3 antibody-drug conjugate, (b) 0.05 mg/mL to 0.6 mg/mL polysorbate, (c) 10 mg/mL to 150 mg/mL sugar, and (d) 1 mM to 50 mM acetate buffer; the pharmaceutical composition has a pH of 4.9-5.5.

In an optional embodiment, provided is the pharmaceutical composition according to any one of the foregoing, which comprises:

(a) on a protein basis, 1 mg/mL to 40 mg/mL said anti-HER3 antibody-drug conjugate, (b) 0.05 mg/mL to 0.6 mg/mL polysorbate, (c) 10 mg/mL to 150 mg/mL sugar, and (d) 1 mM to 50 mM acetate buffer; the pharmaceutical composition has a pH of 5.0-5.5.

In an optional embodiment, provided is the pharmaceutical composition according to any one of the foregoing, which comprises:

(a) on a protein basis, 1 mg/mL to 40 mg/mL said anti-HER3 antibody-drug conjugate, (b) 0.05 mg/mL to 0.6 mg/mL polysorbate, (c) 40 mg/mL to 120 mg/mL sugar, and (d) 1 mM to 50 mM acetate buffer; the pharmaceutical composition has a pH of 5.0-5.5.

In an optional embodiment, provided is the pharmaceutical composition according to any one of the foregoing, which comprises:

(a) 10 mg/mL to 30 mg/mL said anti-HER3 antibody-drug conjugate, (b) 0.1 mg/mL to 0.4 mg/mL polysorbate, (c) 40 mg/mL to 120 mg/mL sugar, and (d) 5 mM to 15 mM acetate buffer; the pharmaceutical composition has a pH of 5.0-5.5.

In an optional embodiment, provided is the pharmaceutical composition according to any one of the foregoing, which comprises:

(a) on a protein basis, 10 mg/mL to 30 mg/mL said anti-HER3 antibody-drug conjugate, (b) 0.1 mg/mL to 0.4 mg/mL polysorbate, (c) 60 mg/mL to 120 mg/mL sugar, and (d) 5 mM to 15 mM acetate buffer; the pharmaceutical composition has a pH of 5.0-5.5.

In an optional embodiment, provided is the pharmaceutical composition according to any one of the foregoing,

which comprises:
(a) on a protein basis, 15 mg/mL to 25 mg/mL said anti-HER3 antibody-drug conjugate, (b) 0.1 mg/mL to 0.4 mg/mL polysorbate 80, (c) 40 mg/mL to 80 mg/mL sucrose, and (d) 5 mM to 15 mM acetic acid-sodium acetate buffer; the pharmaceutical composition has a pH of 5.1-5.3.

In an optional embodiment, provided is the pharmaceutical composition according to any one of the foregoing, which comprises:
(a) on a protein basis, 10 mg/mL to 30 mg/mL said anti-HER3 antibody-drug conjugate, (b) 0.1 mg/mL to 0.4 mg/mL polysorbate, (c) 40 mg/mL to 80 mg/mL sugar, and (d) 5 mM to 15 mM acetate buffer; the pharmaceutical composition has a pH of 5.0-5.5.

In an optional embodiment, provided is the pharmaceutical composition according to any one of the foregoing, which comprises:
(a) on a protein basis, 20 mg/mL to 40 mg/mL said anti-HER3 antibody-drug conjugate, (b) 0.2 mg/mL to 0.4 mg/mL polysorbate 80, (c) 60 mg/mL to 120 mg/mL sucrose, and (d) 10 mM to 20 mM acetic acid-sodium acetate buffer; the pharmaceutical composition has a pH of 4.9-5.5.

In an optional embodiment, provided is the pharmaceutical composition according to any one of the foregoing, which comprises:
(a) on a protein basis, about 20 mg/mL said anti-HER3 antibody-drug conjugate, (b) about 0.4 mg/mL polysorbate 80, (c) about 120 mg/mL sugar, and (d) about 10 mM acetic acid-sodium acetate buffer; the pharmaceutical composition has a pH of about 5.2.

In an optional embodiment, provided is the pharmaceutical composition according to any one of the foregoing, which comprises:
(a) on a protein basis, about 20 mg/mL said anti-HER3 antibody-drug conjugate, (b) about 0.2 mg/mL polysorbate 80, (c) about 60 mg/mL sucrose, and (d) about 10 mM acetic acid-sodium acetate buffer; the pharmaceutical composition has a pH of about 5.2.

In an optional embodiment, the pharmaceutical composition according to any one of the foregoing is a liquid formulation. The liquid formulation or the reconstituted formulation of the present disclosure has relatively good stability. Further, stable liquid formulations include liquid formulations that exhibit desirable features after one month of storage at temperatures including 40 °C.

The present disclosure further provides a freeze-dried formulation comprising an antibody-drug conjugate, wherein the formulation is capable of being reconstituted to form the pharmaceutical composition described above; preferably, the reconstitution is performed with water for injection.

The present disclosure further provides a method for preparing a freeze-dried formulation comprising an antibody-drug conjugate, the method comprising a step of lyophilizing the pharmaceutical composition described above.

In an optional embodiment, the lyophilization in the method for preparing a freeze-dried formulation comprising an antibody-drug conjugate comprises steps of pre-freezing, primary drying, and secondary drying in sequence. The lyophilization is performed by freezing the formulation and subsequently sublimating water at a temperature suitable for primary drying. Under these conditions, the product is at a temperature lower than the eutectic point or the collapse temperature of the formulation. Typically, the primary drying temperature ranges from about -30 °C to 25 °C (assuming the product remains frozen during primary drying). The formulation, the size and type of the container (e.g., glass vial) containing the sample, and the liquid volume determine the time required for drying, which may range from a few hours to several days (e.g., 40-60 hours). The secondary drying may be performed at about 0 °C-40 °C, mainly depending on the type and size of the container and the type of the protein used. The time needed for the secondary drying is determined by the desired residual water content in the product, and it typically takes at least about 5 hours. Generally, the water content of a formulation lyophilized at low pressure is less than about 5%, preferably less than about 3%. The pressure may be the same as that applied to the step of primary drying; preferably, the pressure of secondary drying is lower than that of primary drying. The conditions for lyophilization may vary depending on the formulation and the vial size.

In an optional example of the present disclosure, a lyophilization program is as follows:

(1) pre-freezing for 0.5 h to 1 h in a -2 °C to -8 °C environment;
(2) pre-freezing for 120-180 min in a -45 °C environment;
(3) drying for 2100-2700 min in a -15 °C to -20 °C, 0.1 mbar environment; and
(4) drying for 240-480 min in a 25 °C, 0.05 mbar environment.

In some embodiments, the freeze-dried formulation is stable at 40 °C for at least 7 days, at least 14 days, at least 28 days, or at least 30 days.

The present disclosure further provides a freeze-dried formulation comprising an antibody-drug conjugate, wherein the freeze-dried formulation is obtained by lyophilizing the pharmaceutical composition of the antibody-drug conjugate described above.

The present disclosure further provides a reconstituted solution comprising an antibody-drug conjugate, wherein the reconstituted solution is prepared by reconstituting the freeze-dried formulation described above.

The present disclosure further provides a method for preparing the reconstituted solution described above, the method comprising a step of reconstituting the aforementioned freeze-dried formulation with a solution selected from the group consisting of, but not limited to, water for injection, normal saline, and glucose solution.

In some embodiments, provided is the pharmaceutical composition or the reconstituted solution according to any one of the above, which is a formulation for intravenous infusion, a formulation for intravenous injection, a formulation for subcutaneous injection, a formulation for intraperitoneal injection, or a formulation for intramuscular injection. In some embodiments, provided is the pharmaceutical composition or the reconstituted solution according to any one of the above, which is a formulation for intravenous infusion.

In some embodiments, provided is the pharmaceutical composition or the reconstituted solution according to any one of the above, which is suitable for intravenous infusion, intravenous injection, subcutaneous injection, intraperitoneal injection, or intramuscular injection, preferably for intravenous infusion.

In some embodiments, provided is the pharmaceutical composition or the reconstituted solution or the freeze-dried formulation according to any one of the above, which is used for preparing a medicament for intravenous infusion, intravenous injection, subcutaneous injection, intraperitoneal injection, or intramuscular injection, preferably for preparing a medicament for intravenous infusion.

The present disclosure further provides a product comprising a container containing the pharmaceutical composition, the freeze-dried formulation, or the reconstituted solution described above. In some embodiments, the container is a tubular injection vial made of neutral borosilicate glass.

The present disclosure further provides use of the aforementioned pharmaceutical composition or freeze-dried formulation or reconstituted solution or product in the preparation of a medicament for treating or preventing a tumor.

The present disclosure further provides a method for treating a disease, the method comprising administering to a patient in need thereof the aforementioned pharmaceutical composition or freeze-dried formulation or reconstituted solution or product.

The present disclosure provides a pharmaceutical composition of an anti-HER3 antibody, wherein the anti-HER3 antibody comprises a heavy chain variable region and a light chain variable region; the heavy chain variable region comprises a HCDR1, a HCDR2, and a HCDR3 set forth in SEQ ID NO: 1, SEQ ID NO: 2, and SEQ ID NO: 3, respectively, and the light chain variable region comprises a LCDR1, a LCDR2, and a LCDR3 set forth in SEQ ID NO: 4, SEQ ID NO: 5, and SEQ ID NO: 6, respectively;

the pharmaceutical composition further comprises an acetate buffer, preferably an acetic acid-sodium acetate buffer;
the pharmaceutical composition has a pH of 5.0 to 5.5;
the antibody is at a concentration of 1 mg/mL to 40 mg/mL;
the sugar is at a concentration of 10 mg/mL to 100 mg/mL; and
the acetate buffer is at a concentration of 1 mM to 50 mM.

In an optional embodiment, provided is the pharmaceutical composition of the anti-HER3 antibody according to any one of the foregoing, wherein the anti-HER3 antibody comprises a heavy chain variable region and a light chain variable region, wherein:
the amino acid sequence of the heavy chain variable region is set forth in SEQ ID NO: 7, and the amino acid sequence of the light chain variable region is set forth in SEQ ID NO: 8.

In an optional embodiment, provided is the pharmaceutical composition of the anti-HER3 antibody according to any one of the foregoing, wherein the anti-HER3 antibody comprises a heavy chain set forth in SEQ ID NO: 9 and a light chain set forth in SEQ ID NO: 10.

In an optional embodiment, the pharmaceutical composition of the anti-HER3 antibody according to any one of the foregoing further comprises a sugar. As used herein, "sugar" includes the general composition $(CH_2O)_n$ and derivatives thereof, including monosaccharides, disaccharides, trisaccharides, polysaccharides, sugar alcohols, reducing sugars, non-reducing sugars, etc. The sugar may be selected from the group consisting of glucose, sucrose, trehalose, $\alpha,\alpha$-trehalose dihydrate, lactose, fructose, maltose, dextran, glycerin, erythritol, glycerol, arabitol, sylitol, sorbitol, mannitol, melibiose, melezitose, raffinose, mannotriose, stachyose, maltose, lactulose, maltulose, glucitol, maltitol, lactitol, iso-maltulose, etc. Preferably, the sugar is a non-reducing disaccharide; more preferably, the sugar is $\alpha,\alpha$-trehalose dihydrate or sucrose; most preferably, the sugar is sucrose.

In an optional embodiment, the sugar in the pharmaceutical composition of the anti-HER3 antibody according to any one of the foregoing is at a concentration of 10 mg/mL to 100 mg/mL, preferably 40 mg/mL to 80 mg/mL; non-limiting examples include 10 mg/mL, 25 mg/mL, 30 mg/mL, 35 mg/mL, 40 mg/mL, 45 mg/mL, 50 mg/mL, 55 mg/mL, 60 mg/mL, 70 mg/mL, 80 mg/mL, 90 mg/mL, and 100 mg/mL, as well as any range between these point values; preferably, the concentration is 60 mg/mL to 90 mg/mL; more preferably, the concentration is about 60 mg/mL. The term "about" optimally

indicates ±10%; for example, the concentration of sucrose is about 60 mg/mL, i.e., 60 mg/mL ± 6 mg/mL.

In an optional embodiment, the buffer in the pharmaceutical composition of the anti-HER3 antibody according to any one of the foregoing is at a concentration of about 1 mM to about 50 mM; non-limiting examples include 1 mM, 5 mM, 8 mM, 9 mM, 10 mM, 11 mM, 12 mM, 15 mM, 16 mM, 17 mM, 18 mM, 19 mM, 20 mM, 30 mM, 40 mM, and 50 mM, as well as any range between these point values; preferably, the concentration is 5 mM to 15 mM; more preferably, the concentration is about 10 mM. The term "about" optimally indicates ±10%; for example, the concentration of the acetic acid-sodium acetate buffer is about 10 mM, i.e., 10 mM ± 1 mM.

In an optional embodiment, the anti-HER3 antibody in the pharmaceutical composition of the anti-HER3 antibody according to any one of the foregoing is at a concentration of 1 mg/mL to 40 mg/mL; non-limiting examples include 1 mg/mL, 10 mg/mL, 11 mg/mL, 12 mg/mL, 13 mg/mL, 14 mg/mL, 15 mg/mL, 16 mg/mL, 17 mg/mL, 18 mg/mL, 19 mg/mL, 20 mg/mL, 21 mg/mL, 22 mg/mL, 23 mg/mL, 24 mg/mL, 25 mg/mL, 26 mg/mL, 27 mg/mL, 28 mg/mL, 29 mg/mL, 30 mg/mL, and 40 mg/mL, as well as any range between these point values; preferably, the anti-HER3 antibody is at a concentration of 10 mg/mL to 30 mg/mL; more preferably, the anti-HER3 antibody is at a concentration of 18 mg/mL to 22 mg/mL; most preferably, the anti-HER3 antibody is at a concentration of about 20 mg/mL. Specifically, non-limiting examples include 20.1 mg/mL, 20.2 mg/mL, 20.3 mg/mL, 20.4 mg/mL, 20.5 mg/mL, 20.6 mg/mL, 20.7 mg/mL, 20.8 mg/mL, 20.81 mg/mL, 20.82 mg/mL, 20.83 mg/mL, 20.84 mg/mL, 20.85 mg/mL, 20.86 mg/mL, 20.87 mg/mL, 20.88 mg/mL, 20.89 mg/mL, 20.9 mg/mL, 20.91 mg/mL, 20.92 mg/mL, 20.93 mg/mL, 20.94 mg/mL, 20.95 mg/mL, 20.96 mg/mL, 20.97 mg/mL, 20.98 mg/mL, 20.99 mg/mL, and 21 mg/mL, as well as any range between these point values. The term "about" optimally indicates ±10%; for example, the protein concentration is optimally about 20 mg/mL, i.e., 20 mg/mL ± 10 mg/mL.

In an optional embodiment, provided is the pharmaceutical composition of the anti-HER3 antibody according to any one of the foregoing, which comprises:

(a) 10 mg/mL to 30 mg/mL anti-HER3 antibody, (b) 60 mg/mL to 90 mg/mL sugar, and (c) 5 mM to 15 mM acetate buffer; the pharmaceutical composition has a pH of 5.1-5.4; In an optional embodiment, provided is the pharmaceutical composition of the anti-HER3 antibody according to any one of the foregoing, which comprises:

(a) 10 mg/mL to 30 mg/mL anti-HER3 antibody, (b) 40 mg/mL to 80 mg/mL sugar, and (c) 5 mM to 15 mM acetate buffer; the pharmaceutical composition has a pH of 5.1-5.4; In an optional embodiment, provided is the pharmaceutical composition of the anti-HER3 antibody according to any one of the foregoing, which comprises:

(a) about 20 mg/mL said anti-HER3 antibody, (b) about 60 mg/mL sucrose, and (c) about 10 mM acetic acid-sodium acetate buffer; the pharmaceutical composition has a pH of about 5.2.

The present disclosure further provides the aforementioned pharmaceutical composition of the anti-HER3 antibody-drug conjugate, or pharmaceutical composition of the anti-HER3 antibody, or freeze-dried formulation, or reconstituted solution, or product, as a medicament for use in treating a disease.

The present disclosure further provides the aforementioned pharmaceutical composition of the anti-HER3 antibody-drug conjugate, or pharmaceutical composition of the anti-HER3 antibody, or freeze-dried formulation, or reconstituted solution, or product, as a medicament for use in treating a tumor or cancer.

In an optional embodiment, the disease is a HER3-mediated disease or disorder.

The present disclosure further provides use of the aforementioned pharmaceutical composition of the anti-HER3 antibody-drug conjugate, or pharmaceutical composition of the anti-HER3 antibody, or freeze-dried formulation, or reconstituted solution, or product as a medicament in the preparation of a medicament for treating a viral infection, tumor, or cancer, wherein the tumor or cancer is selected from the group consisting of breast cancer, non-small cell lung cancer, gastric cancer, ovarian cancer, prostate cancer, bladder cancer, colorectal cancer, head and neck squamous cell carcinoma, and melanoma.

When a point value is referred to in the present disclosure, it should be understood that the point value includes an error range. This error range is due to factors such as laboratory environment, personnel operations, instruments, methodology, measurement errors, and the like.

As is well known to those skilled in the art, one, some, or all of the features of the various embodiments described in the present disclosure may be further combined to form other embodiments of the present disclosure. The above embodiments of the present disclosure and other embodiments obtained by combination are further illustrated through the following detailed description.

## Detailed Description of the Invention

The present disclosure provides a pharmaceutical composition that favors production and administration and is stable in properties. Undesirable instability may include any one or more of aggregation, deamidation (e.g., Asn deamidation), oxidation (e.g., Met oxidation), isomerization (e.g., Asp isomerization), clipping/hydrolysis/fragmentation (e.g., hinge region fragmentation), succinimide formation, unpaired cysteines, dissociation of toxins, and the like. Specifically, the pharmaceutical composition described in the present disclosure comprises an antibody-drug conjugate and a buffer.

## Terminology

In order to facilitate the understanding of the present disclosure, certain technical and scientific terms are specifically defined below. Unless otherwise explicitly defined herein, all other technical and scientific terms used herein have the meanings generally understood by those of ordinary skill in the art to which the present disclosure belongs.
"Antibody-drug conjugate (ADC)" means that an antibody is linked to a biologically active cytotoxin or a small-molecule drug with cell killing activity by a linker unit.
"Drug loading" is also referred to as the drug-to-antibody ratio (DAR), i.e., the average number of drugs conjugated per antibody in the ADC. It may range, for example, from about 1 to about 10 drugs conjugated per antibody, and in certain embodiments, from about 1 to about 8 drugs conjugated per antibody; preferably, it is selected from the group consisting of 2-8, 2-7, 2-6, 2-5, 2-4, 3-4, 3-5, 5-6, 5-7, 5-8, and 6-8. Illustratively, the drug loading may be an average of 1, 2, 3, 4, 5, 6, 7, 8, 9, or 10. The ADC general formula of the present disclosure includes a set of antibodies conjugated to drugs within a certain range as described above. In an embodiment of the present disclosure, the drug loading may be represented by n. The drug loading can be determined by conventional methods such as UV/visible spectroscopy, mass spectrometry, ELISA, and HPLC.
The term "linker unit" or "linker fragment" refers to a chemical structure fragment or bond that is linked to an antibody or an antigen-binding fragment thereof at one end and to a drug at the other end, and it may also be linked to a drug after being linked to another linker.
The linker comprises a stretcher, a spacer, and an amino acid unit and may be synthesized using methods known in the art, such as those described in US20050238649A1. The linker may be a "cleavable linker" favorable for the release of drugs in cells. For example, acid-labile linkers (e.g., hydrazones), protease-sensitive (e.g., peptidase-sensitive) linkers, photolabile linkers, dimethyl linkers or disulfide-containing linkers may be used (Chari et al., Cancer Research, 52:127-131 (1992); U.S. Patent No. US5208020A).
The term "drug linker arm fragment" or "drug-linker fragment" refers to a fragment formed by linking a drug and a linker unit, which can be linked to an antibody through the other end of the linker unit.
The loading of the cytotoxic drug can be controlled using the following non-limiting methods, including:

(1) controlling the molar ratio of the drug linker arm fragment to the monoclonal antibody,
(2) controlling reaction time and temperature, and
(3) selecting different reaction reagents.

The three-letter and single-letter codes for amino acids used in the present disclosure are as described in J. Biol. Chem., 243, p3558 (1968).
As used herein, "antibody" is used in the broadest sense and encompasses a variety of antibody structures, including but not limited to full-length antibodies and antibody fragments (or antigen-binding fragments, or antigen-binding moieties), as long as they exhibit the desired antigen-binding activity. In general, a natural intact antibody is a four-peptide-chain structure formed by linking two identical heavy chains and two identical light chains by interchain disulfide bonds.
The engineered antibodies or antigen-binding fragments of the present disclosure can be prepared and purified by conventional methods. For example, cDNA sequences encoding the heavy and light chains can be cloned and recombined into a GS expression vector. Recombinant immunoglobulin expression vectors can be stably transfected into CHO cells. As a more recommended prior art, mammalian expression systems will result in glycosylation of antibodies, particularly at the highly conservative N-terminal site of the Fc region. Positive clones are expanded in a serum-free culture medium in a bioreactor to produce antibodies. The culture medium with the secreted antibody can be purified by conventional techniques. For example, purification is performed using an A or G Sepharose FF column containing an adjusted buffer. Non-specifically bound fractions are washed away. The bound antibody is eluted by the pH gradient method, and the antibody fragments are detected by SDS-PAGE and collected. The antibodies can be filtered and concentrated by conventional methods. Soluble mixtures and polymers can also be removed by conventional methods, such as molecular sieves and ion exchange. The resulting products need to be immediately frozen, such as at -70 °C, or lyophilized.
"Buffer" refers to a buffer that resists changes in pH by the action of its acid-base conjugate components. Examples of buffers that maintain the pH within an appropriate range include acetate, succinate, gluconate, histidine, oxalate, lactate, phosphate, citrate, citric acid-sodium citrate, tartrate, fumarate, glycylglycine, and other organic acid buffers. "Histidine buffer" is a buffer comprising histidine. Examples of histidine buffers include histidine-histidine hydrochloride, histidine-histidine acetate, histidine-histidine phosphate, histidine-histidine sulfate, and the like. The histidine-histidine hydrochloride buffer is preferred. The histidine-histidine hydrochloride buffer may be prepared from histidine and hydrochloric acid, or from histidine and histidine hydrochloride.
"Citrate buffer" is a buffer comprising citrate ions. Examples of citrate buffers include citric acid-sodium citrate, citric acid-potassium citrate, citric acid-calcium citrate, citric acid-magnesium citrate, and the like. The preferred citrate

buffer is citric acid-sodium citrate.

"Succinate buffer" is a buffer comprising succinate ions. Examples of succinate buffers include succinic acid-sodium succinate, succinic acid-potassium succinate, succinic acid-calcium succinate, and the like. The preferred succinate buffer is succinic acid-sodium succinate. Illustratively, the succinic acid-sodium succinate may be prepared from succinic acid and sodium hydroxide, or from succinic acid and sodium succinate.

"Phosphate buffer" is a buffer comprising phosphate ions. Examples of phosphate buffers include disodium hydrogen phosphate-sodium dihydrogen phosphate, disodium hydrogen phosphate-potassium dihydrogen phosphate, disodium hydrogen phosphate-citric acid, and the like. The preferred phosphate buffer is disodium hydrogen phosphate-sodium dihydrogen phosphate.

"Acetate buffer" is a buffer comprising acetate ions. Examples of acetate buffers include acetic acid-sodium acetate, histidine-histidine acetate, acetic acid-potassium acetate, acetic acid-calcium acetate, acetic acid-magnesium acetate, and the like. The preferred acetate buffer is acetic acid-sodium acetate.

"Pharmaceutical composition" refers to a mixture containing one or more of the antibody-drug conjugates described herein or physiologically/pharmaceutically acceptable salts or prodrugs thereof, and other chemical components; the other components are, for example, physiologically/pharmaceutically acceptable carriers and excipients. The purpose of the pharmaceutical composition is to maintain the stability of the active ingredient of the antibody and promote the administration to an organism, which facilitates the absorption of the active ingredient, thereby exerting biological activity.

As used herein, "pharmaceutical composition" and "formulation" are not mutually exclusive.

Unless otherwise stated, the solvent in the pharmaceutical composition described herein in solution form is water.

"Freeze-dried formulation" refers to a formulation or a pharmaceutical composition obtained by lyophilizing a pharmaceutical composition or a formulation in liquid or solution form *in vacuo.*

It should be understood by those skilled in the art that when used with reference to numerical ranges, cutoff values, or specific values, "about" may mean within 1 or more than 1 standard deviation. Alternatively, "about" may mean a range up to 20% apart (i.e., ±20%). Since many of the numerical values used herein are determined experimentally, those skilled in the art will appreciate that such determinations can and typically do vary from experiment to experiment. Because of this inherent difference, it is believed that the values used herein should not be unduly limited. Thus, the term "about" is used to encompass variations of ±20% or less, variations of ±10% or less, variations of ±5% or less, variations of ±1% or less, variations of ±0.5% or less, or variations of ±0.1% or less from a specified value.

The pharmaceutical composition of the present disclosure can achieve a stable effect: a pharmaceutical composition in which the antibody-drug conjugate substantially retains its physical and/or chemical stability and/or biological activity after storage; preferably, the pharmaceutical composition substantially retains its physical and chemical stability as well as its biological activity after storage. The storage period is generally selected based on a predetermined shelf life of the pharmaceutical composition. There are a variety of analytical techniques currently available for measuring the stability of a protein or an antibody-drug conjugate, and the stability after storage for a selected period of time at a selected temperature can be measured.

A stable formulation is one in which no significant change is observed under the following conditions: stored at refrigeration temperature (2-8 °C) for at least 6 months, preferably 12 months, and more preferably 2 years. In addition, stable liquid formulations include liquid formulations that exhibit desirable features after storage at temperatures including 25 °C for periods of time including 1 month, 3 months, and 6 months. Further, stable liquid formulations include liquid formulations that exhibit desirable features after storage at temperatures including 40 °C for periods of time including 10 days, 20 days, and 1 month. Typical examples for stability are as follows: generally, no more than about 10%, preferably no more than about 5%, of antibody monomers aggregate or are degraded as measured by SEC-HPLC. The formulation is a pale yellow, nearly colorless and clear liquid, or colorless, or clear to slightly opalescent, by visual analysis. The concentration, pH, and osmolality of the formulation have a change of no more than ±10%. Generally, a decrease of no more than about 10%, preferably no more than about 5% is observed. Generally, aggregation of no more than about 10%, preferably no more than about 5% is formed.

An antibody-drug conjugate "retains its physical stability" in a pharmaceutical formulation if it shows no significant increase in aggregation, precipitation, and/or denaturation upon visual examination of color and/or clarity, or as measured by UV light scattering, size exclusion chromatography (SEC), and dynamic light scattering (DLS). Changes in protein conformation can be evaluated by fluorescence spectroscopy (which determines the protein tertiary structure) and by FTIR spectroscopy (which determines the protein secondary structure).

An antibody-drug conjugate "retains its chemical stability" in a pharmaceutical formulation if it shows no significant chemical change. Chemical stability can be evaluated by detecting and quantifying chemically changed proteins. Degradation processes that often change the chemical structure of proteins include hydrolysis or clipping (evaluated by methods such as size exclusion chromatography and CE-SDS), oxidation (evaluated by methods such as peptide mapping in conjunction with mass spectroscopy or MALDI/TOF/MS), deamidation (evaluated by methods such as

ionexchange chromatography, capillary isoelectric focusing, peptide mapping, and isoaspartic acid measurement), and isomerization (evaluated by measuring the isoaspartic acid content, peptide mapping, etc.).

An antibody-drug conjugate "retains its biological activity" in a pharmaceutical formulation if the biological activity of the antibody-drug conjugate at a given time is within a predetermined range of the biological activity exhibited at the time the pharmaceutical formulation was prepared.

"Optional" or "optionally" means that the event or circumstance subsequently described may, but does not necessarily, occur, and this description includes instances where the event or circumstance occurs or does not occur. For example, "optionally comprising 1-3 antibody heavy chain variable regions" means that the antibody heavy chain variable region of a particular sequence may, but does not necessarily, exist.

"Substituted" means that one or more, preferably up to 5, and more preferably 1 to 3, hydrogen atoms in the group are independently substituted with a corresponding number of substituents. It goes without saying that a substituent is only in its possible chemical position, and those skilled in the art will be able to determine (experimentally or theoretically) possible or impossible substitution without undue effort. For example, it may be unstable when an amino or hydroxy having free hydrogen is bound to a carbon atom having an unsaturated (e.g., olefinic) bond.

For the preparation of conventional pharmaceutical compositions, refer to Chinese Pharmacopoeia.

The term "carrier" for the drug of the present disclosure refers to a system that can alter how the drug gets into a human body and the distribution of the drug in the human body, control the release rate of the drug, and deliver the drug to a targeted organ. The drug carrier release and targeted system can reduce drug degradation and loss, reduce side effects, and improve bioavailability. For example, polymeric surfactants that can be used as carriers can self-assemble due to their unique amphiphilic structures to form various forms of aggregates; preferred examples are, e.g., micelles, microemulsions, gels, liquid crystals, and vesicles. The aggregates have the capability of encapsulating drug molecules and have good permeability for membranes, and therefore can be used as excellent drug carriers.

"Administer" and "treat", when applied to animals, humans, experimental subjects, cells, tissues, organs, or biological fluids, refer to contact of an exogenous drug, a therapeutic agent, a diagnostic agent, or a composition with the animals, humans, subjects, cells, tissues, organs, or biological fluids. "Administer" and "treat" can refer to, for example, therapeutic, pharmacokinetic, diagnostic, research, and experimental methods. The treatment of cells comprises contacting a reagent with cells and contacting the reagent with a fluid, where the fluid is in contact with the cells. "Administer" and "treat" also refer to treating, e.g., cells, by reagents, diagnosis, binding compositions or by another cell *in vitro* and *ex vivo.* "Treat", when applied to human, veterinary, or research subjects, refers to therapeutic treatment, preventive or prophylactic measures, and research and diagnostic applications.

"Treatment" refers to administering a therapeutic agent, such as a composition comprising any one of the conjugation compounds of the present disclosure, either internally or externally to a patient with one or more disease symptoms on which the therapeutic agent is known to have a therapeutic effect. Typically, the therapeutic agent is administered in an amount effective to alleviate one or more disease symptoms in the patient or population being treated to induce regression of such symptoms or to inhibit the development of such symptoms to any clinically measurable degree. The amount of the therapeutic agent effective to alleviate any specific disease symptom (also referred to as "therapeutically effective amount") may vary depending on a variety of factors, such as the disease state, age and body weight of the patient, and the ability of the drug to produce a desired therapeutic effect in the patient. Whether a disease symptom has been alleviated can be evaluated by any clinical testing methods commonly used by doctors or other health care professionals to evaluate the severity or progression of the symptom. Although an embodiment of the present disclosure (for example, a treatment method or a product) may not be effective in alleviating every target disease symptom, it should alleviate the target disease symptom in a statistically significant number of patients as determined by any statistical testing methods known in the art, such as Student t-test, chi-square test, Mann and Whitney's U test, Kruskal-Wallis test (H test), Jonckheere-Terpstra test, and Wilcoxon test.

"Effective amount" comprises an amount sufficient to ameliorate or prevent a symptom or disorder of a medical disease. An effective amount also means an amount sufficient to allow or facilitate diagnosis. The effective amount for a particular patient or veterinary subject may vary depending on factors such as the disorder to be treated, the general health of the patient, the method and route and dose of administration, and the severity of adverse effects. An effective amount may be the maximum dose or administration regimen to avoid significant side effects or toxic effects.

"Exchange" refers to the exchange of a solvent system that solubilizes an antibody protein or antibody-drug conjugate. For example, a high-salt or hypertonic solvent system comprising the antibody protein or antibody-drug conjugate is exchanged, by physical operations, with a buffer system of a stable formulation, such that the antibody protein or antibody-drug conjugate is present in the stable formulation. The physical operations include, but are not limited to, ultrafiltration, dialysis, or centrifugation.

## DETAILED DESCRIPTION

The present disclosure is further described below with reference to examples, which, however, are not intended

to limit the scope of the present disclosure. The experimental methods in the examples of the present disclosure in which specific conditions are not specified are generally performed under conventional conditions, for example, by referring to Antibodies: A Laboratory Manual and Molecular Cloning: A Laboratory Manual by Cold Spring Harbor Laboratory, or under conditions recommended by the manufacturer of the raw material or the goods. Reagents without specific origins indicated were commercially available conventional reagents.

**Preparation Example I. Preparation of Antibody**

The HER3 antibody of the present disclosure was derived from the antibody HER3-29 of WO2022078425A1 (incorporated herein by reference in its entirety), and the related sequences are as follows:
1) The CDR sequences of the anti-HER3 antibody are shown in the table below:

Table 1. CDR sequences obtained by the Kabat numbering scheme

| Antibody | HER3-29 | Sequence No. |
|---|---|---|
| Heavy chain CDR1 | DYAMH | SEQ ID NO: 1 |
| Heavy chain CDR2 | GISWNSGSIGYADSVKG | SEQ ID NO: 2 |
| Heavy chain CDR3 | EGLPGLDY | SEQ ID NO: 3 |
| Light chain CDR1 | RASQHVGTYLN | SEQ ID NO: 4 |
| Light chain CDR2 | GAANLQS | SEQ ID NO: 5 |
| Light chain CDR3 | QQSYNTPPFS | SEQ ID NO: 6 |

2) The heavy chain variable region and light chain variable region sequences of the anti-HER3 antibody, a fully human antibody molecule, are as follows:

The heavy chain variable region of HER3:

QVQLVQSGGGLVQPGRSLRLSCAASGFTFDDYAMHWVRQAPGKGLEWVSGIS
WNSGSIGYADSVKGRFTISRDNAKNSLYLQMNSLRAEDTALYYCAKEGLPGLD
YWGQGTLVTVSS

SEQ ID NO: 7

The light chain variable region of HER3:

DIQMTQSPSSLSASIGDRATITCRASQHVGTYLNWYQQKPGKTPKLLISGAANL
QSGVPSRFSGSGSGTDFTLTISSLQPEDFATYYCQQSYNTPPFSFGQGTKVEIK

SEQ ID NO: 8

The heavy chain and light chain sequences of the anti-HER3 antibody HER3-29, a fully human antibody molecule, are as follows:

The heavy chain of HER3-29:

QVQLVQSGGGLVQPGRSLRLSCAASGFTFDDYAMHWVRQAPGKGLEWVSGIS
WNSGSIGYADSVKGRFTISRDNAKNSLYLQMNSLRAEDTALYYCAKEGLPGLD
YWGQGTLVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEYK
CKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVM
HEALHNHYTQKSLSLSPGK

SEQ ID NO: 9

The light chain of HER3-29:

DIQMTQSPSSLSASIGDRATITCRASQHVGTYLNWYQQKPGKTPKLLISGAANL
QSGVPSRFSGSGSGTDFTLTISSLQPEDFATYYCQQSYNTPPFSFGQGTKVEIK
RTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC

SEQ ID NO: 10

**Preparation Example II. Preparation of ADCs**

The drug moiety of the conjugates of the present disclosure may be any suitable drug. Particularly suitable drugs are described, for example, in PCT Publication No. WO2020063676A1 (incorporated herein by reference in its entirety).

The compound 2-B of the present disclosure (i.e., the compound 2-B of Example 2 of WO2020063676 A1) is (*R*)-2-cyclopropyl-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-2-hydroxyacetamide, which has the following structure:

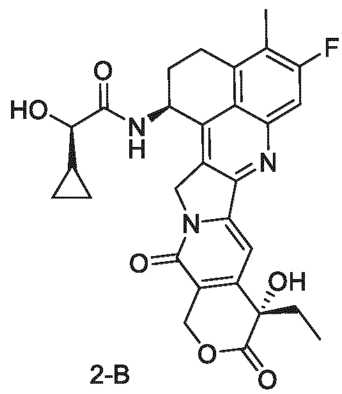

2-B

The compound 9A of the present disclosure (i.e., the compound 9-A of Example 9 of WO2020063676 A1) is N-((2R,105)-10-benzyl-2-cyclopropyl-1-(((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)amino)-1,6,9,12,15-pentaoxo-3-oxa-5,8,11,14-tetraazahexadec-16-yl)-6-(2,5-dioxo-2,5-dihydro-1H-pyrrol-1-yl)hexanamide, which has the following structure:

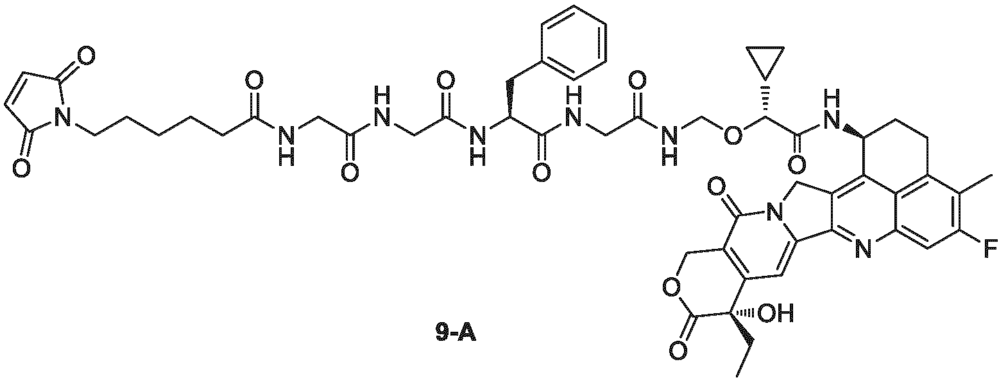

9-A

Exemplary anti-HER3 antibody-drug conjugates of the present disclosure are Example 3-1 (n = 4.19), Example 3-2 (n = 2.91), Example 3-3 (n = 7.27) in WO2022078425A1, which have the following structure:

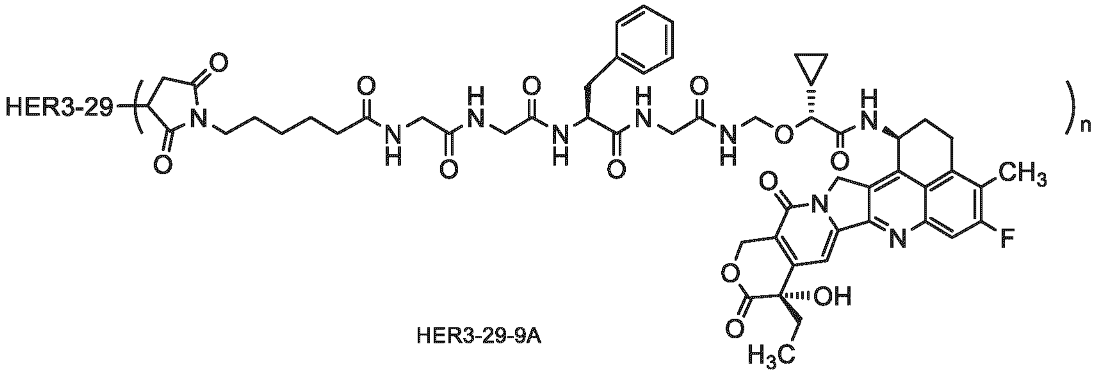

HER3-29-9A

where n is 1 to 8, preferably 3 to 5.

The molecular structure of the formulation conjugate in the present disclosure is abbreviated as the following structural formula ADC-1:

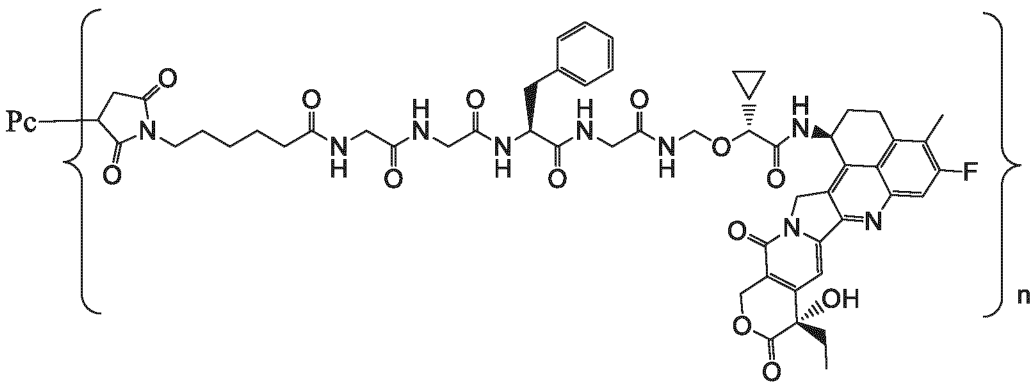

The best example n (DAR value) of the formulations in the present disclosure is about 4.0, which is equivalent to 4.0 ± 0.4.

## Preparation Example III. Preparation of Formulations

The equipment and parameters used in the formulation preparation and analysis and the calculation methods for the results are shown below:

1) SEC-HPLC purity:

According to the molecular sieve mechanism of a gel chromatography column, size-exclusion chromatography

(SEC) was used to separate monomers and aggregates based on the molecular size of a test component. At a detection wavelength of 280 nm, the proportions of monomers and aggregates were calculated using the area normalization method.

Instrument for SEC analysis: Agilent 1260 high performance liquid chromatograph. Column: Tosoh, TSKgel G3000SWXL (7.8 mm × 30 cm, 5 μm).

2) CE-SDS purity (reduced CE-SDS and non-reduced CE-SDS purity):

The reduced CE-SDS and non-reduced CE-SDS purity was determined by capillary gel electrophoresis. This method uses a capillary as the separation channel and uses a high-voltage direct current electric field as the driving force to achieve separation based on differences in the mobility (migration speed under a unit electric field strength) and (or) partitioning behavior of the components within a sample.

Instrument for CE-SDS analysis: capillary electrophoresis system, model: Beckman/ PA800 plus.

△R-CE% refers to the difference between the value of this test item after sample placement under various conditions and the value at the start of placement.

3) Protein concentration determination:

In the present disclosure, the concentration of an antibody-drug conjugate is calculated based on the protein, i.e., expressed in terms of the concentration of the antibody moiety in the antibody-drug conjugate.

Since the toxin in the antibody-drug conjugate shows absorption both at the characteristic absorption wavelength of the protein of 280 nm and at 370 nm, the following formula was used to calculate the above protein concentration:

$$A_{280nm} = \left(C_{drug} \times E_{drug-280} + C_{mAb} \times E_{mAb-280}\right) \times l$$
$$A_{370nm} = C_{drug} \times E_{drug-370} \times l$$

Let

$$R = \frac{E_{drug-370}}{E_{drug-280}}$$

Namely:

$$C_{mAb} = \frac{R \times A_{280nm} - A_{370nm}}{R \times E_{mAb-280}}$$

In the formula, $A_{280nm}$: the average absorbance of a single sample of the test sample solution at an optical path length of 1 cm and a wavelength of 280 nm;

$A_{370nm}$: the average absorbance of a single sample of the test sample solution at an optical path length of 1 cm and a wavelength of 370 nm;
$E_{mAb-280}$: the mass extinction coefficient of the protein at a wavelength of 280 nm, which is 1.532 $g^{-1}cm^{-1}L$;
$E_{drug-280}$: the mass extinction coefficient of the toxin at a wavelength of 280 nm, which is 5.17 $g^{-1}cm^{-1}L$;
$E_{drug-370}$: the mass extinction coefficient of the toxin at a wavelength of 370 nm, which is 17.89 $g^{-1}cm^{-1}L$;
R: the ratio of the extinction coefficient of the toxin at 370 nm to that at 280 nm, which is 3.46;
$C_{mAb}$: the concentration of the protein, mg/mL;
l: the optical path length, cm (here the optical path length is 1 cm).

If the test solution is diluted, the protein concentration is: C (mg/mL) = $C_{mAb}$ × N, and N is the dilution factor.

Instrument for protein concentration determination: ultraviolet-visible spectrophotometer, model: Nano Drop 2000.

4) Appearance:

Visual inspection was used. The sample vial was wiped clean, and the appearance of the freeze-dried powder

cake was directly observed. Under a light intensity of 1500 lx, the color, clarity, and visible foreign objects of the sample solution was observed against a white background and a black background using a clarity analyzer.

Instrument for appearance examination: Jingtuo Instrument YB-2A clarity analyzer.

5) Thermal decomposition onset temperature (Tonset) and particle size (Radius):

The thermal decomposition onset temperature (Tonset) and particle size (Radius) of the protein were determined using a high-throughput dynamic light scattering instrument. A sample was added to a sample plate, and the particle size and dispersion coefficient (%PD) were determined; Tonset was determined with a thermal ramp-up run from 25 °C to 95 °C.

Instrument for Tonset and Radius determination: high-throughput dynamic light scattering instrument, model DynaPro plate ReaderIII.

6) Charge variant content:

The charge variant content was determined by iCIEF analysis. The method uses a capillary as the separation channel and applies direct current voltage to both ends of the capillary. Within the capillary, an ampholyte solution forms a certain range of pH gradient. Components migrate to their respective isoelectric points based on their charge differences, focusing into very narrow sections. As a result, the components are separated. Instrument for iCIEF analysis: Protein Simple whole-column imaged capillary isoelectric focusing electrophoresis system, model iCE3 or Maurice.

7) DAR value:

The drug antibody ratio (DAR) was determined by hydrophobic interaction chromatography (HIC). This method separates a sample based on the fact that the hydrophobic interactions between a hydrophobic chromatography column and components with different DAR values are different and on the hydrophobic capacity of a test component. At a detection wavelength of 280 nm, after the peak area of each peak was corrected, the DAR value was obtained using the calculation formula.

Instrument for HIC analysis: Agilent 1260 high performance liquid chromatograph. Chromatography column: BioCore HIC-Butyl.

8) Free toxin:

After the protein was precipitated, the supernatant was taken for UPLC-UV analysis. Instrument for analysis: Waters ultra high performance liquid chromatograph, model ACQUITY H-Class.

Exemplary antibody pharmaceutical composition (formulation) preparation process

Step 1: The HER3 antibody-drug conjugate ADC-1 (with a DAR value of $4.0 \pm 0.4$) and a stabilizer were prepared into a formulation stock solution containing the ADC. The stock solution was filtered through a 0.22 μm filter to remove bacteria, and the filtrate was collected.

Step 2: The filling volume was adjusted (the target filling volume was 5.3 mL/vial), and vials were filled with the collected filtrate. At the start of filling, during filling, and at the end of filling, samples were taken and examined for filling volume difference.

Step 3: Half plugging, lyophilization, and full plugging were performed, and a capping machine was started for capping.

Step 4: Visual inspection was performed to confirm that the products do not have appearance defects such as powder cake collapse, shrinking, and melting. Carton labels were printed. Cartons were folded. The products were packed in the cartons. The carton labels were put on the cartons.

The lyophilization process for ADC formulations was as follows:

(1) pre-freezing for 0.5-1 h in a -2 °C to -8 °C environment;
(2) pre-freezing for 120-180 min in a -45 °C environment;
(3) drying for 2100-2700 min in a -15 °C to -20 °C, 0.1 mbar environment; and
(4) drying for 240-480 min in a 25 °C, 0.05 mbar environment.

**Example 1. pH Screening for Antibody HER3-29**

A 10 mM acetic acid-sodium acetate buffer system was selected, a total of 6 different pH values, 3.7, 4.6, 5.0, 5.4,

5.8, and 6.2, were designed, and a total of 6 formulas with an antibody HER3-29 concentration of 25.0 mg/mL were prepared. The thermal decomposition onset temperature (Tonset) and the particle size (Radius) of the samples were determined, and the stability of the protein under different pH conditions was assessed.

1) 10 mM acetic acid-sodium acetate, pH 3.7;
2) 10 mM acetic acid-sodium acetate, pH 4.6;
3) 10 mM acetic acid-sodium acetate, pH 5.0;
4) 10 mM acetic acid-sodium acetate, pH 5.4;
5) 10 mM acetic acid-sodium acetate, pH 5.8;
6) 10 mM acetic acid-sodium acetate, pH 6.2.

Table 2. The results of pH screening for antibody HER3-29

| No. | pH | Radius (nm) | Tonset (°C) |
|---|---|---|---|
| 1 | 3.7 | 4.2 | 44.20 |
| 2 | 4.6 | 5.4 | 53.48 |
| 3 | 5.0 | 4.6 | 58.18 |
| 4 | 5.4 | 7.0 | 57.70 |
| 5 | 5.8 | 7.0 | 58.06 |
| 6 | 6.2 | 7.1 | 58.19 |

The results show that antibody HER3-29 exhibited a relatively small particle size and a relatively high Tonset value at pH 5.0, indicating that the protein is not prone to aggregation at this pH. Given that the pH range is generally the target value ± 0.2 and that the Tonset value was higher when pH > 5.0 compared to when the pH was less than 5.0, the preferred pH for antibody HER3-29 is 5.2 ± 0.2.

**Example 2. pH Confirmation for Antibody HER3-29**

A pH 5.2, 10 mM acetic acid-sodium acetate buffer system was selected, and a formula sample with an antibody HER3-29 concentration of 20 mg/mL was prepared. The stability of the formula under high temperature (40 °C), 25 °C, 2-8 °C, -35 °C, and repeated freeze-thaw (-35 °C and room temperature) conditions was assessed based on the appearance and the SEC-HPLC and iCIEF purity of the sample.
10 mM acetic acid-sodium acetate, pH 5.2, 20 mg/mL antibody HER3-29.

Table 3. Results 1 of pH confirmation for antibody HER3-29

| Appearance | | | | | | | | | |
|---|---|---|---|---|---|---|---|---|---|
| T0 | -35 °C | | 2~8 °C | | 25 °C | | 40 °C | Freeze-thaw | |
| | 1M | 3M | 1M | 3M | 1W | 4W | 1W | 3 cycles | 5 cycles |
| Colorless, clear, a large number of protein particles | Colorless, clear, an extremely large number of fine bubbles | Colorless, clear, an extremely large number of fine bubbles | Colorless, clear, a small number of white particles | Colorless, clear, a large number of white particles | Colorless, clear, a small number of visible particles | Colorless, clear, a small amount of white fibrous material | Colorless, clear, a large amount of white fibrous material | Colorless, slightly opalescent, an extremely large number of fine bubbles | Colorless, slightly opalescent, an extremely large number of fine bubbles |

Table 4. Results 2 of pH confirmation for antibody HER3-29

| SEC analysis | | | | | | | | | | |
|---|---|---|---|---|---|---|---|---|---|---|
| | T0 | -35 °C | | 2~8 °C | | 25 °C | | 40 °C | Freeze-thaw | |
| | | 1M | 3M | 1M | 3M | 1W | 4W | 1W | 3 cycles | 5 cycles |
| Monomer% | 96.9 | 96.4 | 95.0 | 96.7 | 97.1 | 97.0 | 96.2 | 97.6 | 96.4 | 96.0 |
| Aggregate% | 3.1 | 3.6 | 4.8 | 3.2 | 2.8 | 2.9 | 3.2 | 1.5 | 3.5 | 3.8 |
| iCIEF analysis | | | | | | | | | | |
| | T0 | -35 °C | | 2~8 °C | | 25 °C | | 40 °C | Freeze-thaw | |
| | | 1M | 3M | 1M | 3M | 1W | 4W | 1W | 3 cycles | 5 cycles |
| Acidic peak% | 34.8 | 40.7 | 38.1 | 42.9 | 37.9 | 45.1 | 43.6 | 48.0 | 35.6 | 35.4 |
| Main peak% | 63.0 | 57.6 | 59.4 | 55.2 | 60.2 | 53.1 | 54.6 | 50.1 | 62.6 | 62.8 |
| Basic peak% | 2.2 | 1.7 | 2.5 | 1.9 | 1.8 | 1.7 | 1.7 | 1.9 | 1.8 | 1.9 |
| Note: T0 stands for day 0, 1M stands for 1 month, 1W stands for 1 week, and so on. | | | | | | | | | | |

The results show that standing under different conditions, the formula sample in the 10 mM acetic acid-sodium acetate buffer system was colorless; under freeze-thaw conditions, the opalescence was slightly greater. Stored at either 2-8 °C or 25 °C, the sample exhibited no significant changes in aggregate or monomer content. However, at - 35 °C and under freeze-thaw conditions, the sample exhibited a trend of higher aggregate contents. Moreover, at a high temperature of 40 °C, aggregates degraded into part of monomers and fragments. At -35 °C and 2-8 °C, the acidic peak content increased somewhat, and the main peak content and the basic peak content decreased somewhat, but the changes were not significant. The freeze-thaw conditions did not significantly influence the charge heterogeneity of the sample. At -35 °C and under freeze-thaw conditions, the increase in antibody HER3-29 aggregate content was relatively significant. At 2-8 °C and 25 °C, the antibody was relatively stable. Given that an additional stabilizer may influence subsequent coupling reactions, 5.2 was selected as the target pH for antibody HER3-29 without adding an additional stabilizer.

**Example 3. Buffer System Screening for Antibody HER3-29**

Four buffer systems, 10 mM histidine-histidine hydrochloride, 10 mM citric acid-sodium citrate, 10 mM succinic acid-sodium succinate, and 10 mM acetic acid-sodium acetate, pH 5.2, were selected, and formula samples with an antibody HER3-29 concentration of 20 mg/mL were prepared. The stability of the formulas under high temperature (40 °C), 25 °C, 2-8 °C, -35 °C, repeated freeze-thaw (-35 °C and room temperature), shaking (200 rpm, 25 °C), and light exposure (25 °C, 5000 lx) conditions was assessed based on the appearance and the SEC-HPLC and iCIEF purity of the samples.

1) 10 mM histidine-histidine hydrochloride, pH 5.2, 20 mg/mL antibody HER3-29;

2) 10 mM citric acid-sodium citrate, pH 5.2, 20 mg/mL antibody HER3-29;

3) 10 mM succinic acid-sodium succinate, pH 5.2, 20 mg/mL antibody HER3-29;

4) 10 mM acetic acid-sodium acetate, pH 5.2, 20 mg/mL antibody HER3-29.

Table 1. Results 1 of buffer system screening for antibody HER3-29

| No. | Appearance | | | | | | | |
|---|---|---|---|---|---|---|---|---|
| | T0 | 40 °C | 25 °C | 2~8 °C | -35 °C | Freeze-thaw | Shaking | Light exposure |
| | | 4W | 4W | 3M | 3M | 5 cycles | 48 h | 10 d |
| 1 | Colorless, clear, free of visible protein particles | NT | NT | Colorless, clear, free of visible protein particles | Colorless, clear, a large number of fine bubbles | Colorless, clear, a large number of fine bubbles | Colorless, clear, free of visible protein particles | Colorless, clear, a small number of protein particles |
| 2 | Colorless, slightly opalescent, free of visible protein particles | NT | NT | Colorless, slightly opalescent, free of visible protein particles | Colorless, slightly opalescent, a large number of fine bubbles | Colorless, slightly opalescent, a large number of fine bubbles | Colorless, slightly opalescent, free of visible protein particles | Colorless, slightly opalescent, free of visible protein particles |
| 3 | Colorless, clear, free of visible protein particles | NT | NT | Colorless, clear, free of visible protein particles | Colorless, clear, a large number of fine bubbles | Colorless, clear, a large number of fine bubbles | Colorless, clear, free of visible protein particles | Colorless, clear, free of visible protein particles |
| 4 | Colorless, clear, free of visible protein particles | Colorless, clear, free of visible protein particles | Colorless, clear, free of visible protein particles | Colorless, clear, free of visible protein particles | Colorless, clear, a large number of fine bubbles | Colorless, clear, a large number of fine bubbles | Colorless, clear, free of visible protein particles | Colorless, clear, free of visible protein particles |

Table 2. Results 2 of buffer system screening for antibody HER3-29

| No. | SEC | T0 | 40 °C | 25 °C | 2~8 °C | -35 °C | Freeze-thaw | Shaking | Light exposure |
|---|---|---|---|---|---|---|---|---|---|
| | | | 4W | 4W | 3M | 3M | 5 cycles | 48h | 10d |
| 1 | Monomer% | 96.2 | NT | NT | 96.1 | 94.6 | 95.0 | 96.1 | 95.9 |
| | Aggregate% | 3.7 | | | 3.7 | 5.3 | 4.9 | 3.8 | 4.0 |
| 2 | Monomer% | 96.1 | NT | NT | 96.3 | 95.7 | 95.7 | 96.1 | 95.5 |
| | Aggregate% | 3.8 | | | 3.6 | 4.1 | 4.2 | 3.8 | 4.3 |
| 3 | Monomer% | 96.1 | NT | NT | 96.1 | 94.6 | 95.4 | 96.1 | 95.9 |
| | Aggregate% | 3.8 | | | 3.8 | 5.3 | 4.5 | 3.8 | 4.0 |
| 4 | Monomer% | 96.1 | 96.8 | 96.1 | 96.2 | 93.9 | 94.9 | 96.1 | 96.0 |
| | Aggregate% | 3.8 | 2.6 | 3.7 | 3.8 | 6.0 | 5.0 | 3.8 | 3.9 |

Table 3. Results 3 of buffer system screening for antibody HER3-29

| No. | iCIEF | T0 | 40 °C | 25 °C | 2~8 °C | -35 °C | Freeze-thaw | Shaking | Light exposure |
|---|---|---|---|---|---|---|---|---|---|
| | | | 4W | 4W | 3M | 3M | 5 cycles | 48 h | 10d |
| 1 | Acidic peak% | 38.3 | NT | NT | 40.1 | 40.2 | NT | NT | 41.2 |
| | Main peak% | 55.7 | | | 58.5 | 58.4 | | | 52.6 |
| | Basic peak% | 6.0 | | | 1.3 | 1.4 | | | 6.2 |

(continued)

| No. | iCIEF | T0 | 40 °C 4W | 25 °C 4W | 2~8 °C 3M | -35 °C 3M | Freeze-thaw 5 cycles | Shaking 48 h | Light exposure 10d |
|---|---|---|---|---|---|---|---|---|---|
| 2 | Acidic peak% | 28.9 | NT | NT | 29.4 | 28.7 | NT | NT | 31.5 |
| | Main peak% | 44.2 | | | 45.0 | 45.3 | | | 42.4 |
| | Basic peak% | 26.9 | | | 25.7 | 25.9 | | | 26.1 |
| 3 | Acidic peak% | 35.6 | NT | NT | 37.5 | 37.5 | NT | NT | 37.4 |
| | Main peak% | 51.9 | | | 55.4 | 54.5 | | | 50.4 |
| | Basic peak% | 12.5 | | | 7.1 | 8.0 | | | 12.1 |
| 4 | Acidic peak% | 41.0 | 53.4 | 41.4 | 39.6 | 40.3 | 41.0 | 40.9 | 41.7 |
| | Main peak% | 57.5 | 44.2 | 57.0 | 59.2 | 58.3 | 57.6 | 57.7 | 56.8 |
| | Basic peak% | 1.5 | 2.4 | 1.6 | 1.2 | 1.4 | 1.4 | 1.4 | 1.5 |
| Note: NT stands for not tested. In formulas 1-3 under different test conditions, the basic peak content was relatively high, and the main peak content was relatively low. Therefore, the samples were not tested under some test conditions. | | | | | | | | | |

The results show that antibody HER3-29 was colorless in each of the different buffer systems and was slightly opalescent in the 10 mM citric acid-sodium citrate system. Under 2-8 °C, shaking, and light exposure conditions, the aggregate content of these formulas did not significantly increase compared to time 0. However, either at -35 °C or under repeated freeze-thaw conditions, the aggregate content increased. At a high temperature of 40 °C, the aggregate content decreased due to degradation of aggregates into part of monomers and fragments. The basic peak content in the 10 mM histidine-histidine hydrochloride, 10 mM citric acid-sodium citrate, and 10 mM succinic acid-sodium succinate systems was significantly higher than that in the 10 mM acetic acid-sodium acetate buffer system. Moreover, in the 10 mM acetic acid-sodium acetate buffer system, the main peak content decreased to a certain extent under high temperature conditions, and there was no significant change under the other test conditions. Therefore, pH 5.2, 10 mM acetic acid-sodium acetate was selected as the buffer system for antibody HER3-29.

**Example 4. Stabilizer Screening for Antibody HER3-29**

According to the previous screening results, the antibody HER3-29 aggregate content in the pH 5.2, 10 mM acetic acid-sodium acetate buffer system increased either at -35 °C or under repeated freeze-thaw conditions. Before coupling, antibody HER3-29 needs to be frozen at -35 °C. To improve the freezing and freeze-thaw stability of antibody HER3-29, a pH 5.2, 10 mM acetic acid-sodium acetate buffer system was selected, 60 mg/mL sucrose was used as a stabilizer, and 0.2 mg/mL polysorbate 80 was added or not added, and a total of 2 formula samples with an antibody HER3-29 concentration of 20 mg/mL were prepared. The stability of the 2 formulas under high temperature (40 °C), 25 °C, 2-8 °C, -35 °C, repeated freeze-thaw (-35 °C and room temperature), shaking (200 rpm, 25 °C), and light exposure (25 °C, 5000 lx) conditions was assessed based on the appearance and the SEC-HPLC and iCIEF purity of the samples.

1) 10 mM acetic acid-sodium acetate, 60 mg/mL sucrose, pH 5.2, 20 mg/mL antibody HER3-29;

2) 10 mM acetic acid-sodium acetate, 60 mg/mL sucrose, 0.2 mg/mL polysorbate 80, pH 5.2, 20 mg/mL antibody HER3-29.

Table 4. Results 1 of stabilizer screening for antibody HER3-29

| **No.** | **Appearance** | | | | | | | |
|---|---|---|---|---|---|---|---|---|
| | **T0** | **40 °C** | **25 °C** | **2~8 °C** | **-35 °C** | **Freeze-thaw** | **Shaking** | **Light exposure** |
| | | **4W** | **4W** | **3M** | **3M** | **5 cycles** | **48 h** | **10 d** |
| 1 | Colorless, clear, free of visible protein particles | Colorless, clear, free of visible protein particles | Colorless, clear, free of visible protein particles | Colorless, clear, free of visible protein particles | Colorless, clear, a large number of fine bubbles | Colorless, clear, a large number of fine bubbles | Colorless, clear, free of visible protein particles | Colorless, clear, free of visible protein particles |
| 2 | Colorless, clear, free of visible protein particles | Colorless, clear, free of visible protein particles | Colorless, clear, free of visible protein particles | Colorless, clear, free of visible protein particles | Colorless, clear, a large number of fine bubbles | Colorless, clear, a large number of fine bubbles | Colorless, clear, free of visible protein particles | Colorless, clear, free of visible protein particles |

Table 5. Results 2 of stabilizer screening for antibody HER3-29

| No. | SEC | T0 | 40 °C | 25 °C | 2~8 °C | -35 °C | Freeze-thaw | Shaking | Light exposure |
|---|---|---|---|---|---|---|---|---|---|
| | | | 4W | 4W | 3M | 3M | 5 cycles | 48 h | 10 d |
| 1 | Monomer% | 96.0 | 96.8 | 96.2 | 96.1 | 96.1 | 96.0 | 96.0 | 95.9 |
| | Aggregate% | 3.9 | 2.5 | 3.7 | 3.7 | 3.8 | 3.9 | 3.9 | 4.0 |
| 2 | Monomer% | 96.0 | 96.8 | 96.0 | 96.1 | 96.0 | 95.9 | 96.1 | 95.8 |
| | Aggregate% | 4.0 | 2.6 | 3.8 | 3.8 | 3.9 | 4.0 | 3.8 | 4.1 |

Table 6. Results 3 of stabilizer screening for antibody HER3-29

| No. | iCIEF | T0 | 40 °C | 25 °C | 2~8 °C | -35 °C | Freeze-thaw | Shaking | Light exposure |
|---|---|---|---|---|---|---|---|---|---|
| | | | 4W | 4W | 3M | 3M | 5 cycles | 48 h | 10d |
| 1 | Acidic peak% | 40.6 | 54.5 | 42.2 | 40.2 | 40.2 | 40.9 | 40.0 | 42.4 |
| | Main peak% | 58.1 | 43.0 | 56.3 | 58.4 | 58.6 | 57.8 | 58.7 | 56.4 |
| | Basic peak% | 1.3 | 2.5 | 1.5 | 1.4 | 1.2 | 1.3 | 1.3 | 1.2 |
| 2 | Acidic peak% | 41.0 | 55.3 | 42.4 | 40.3 | 40.0 | 40.4 | 40.1 | 42.4 |
| | Main peak% | 57.7 | 42.3 | 56.0 | 58.5 | 58.7 | 58.2 | 58.6 | 56.4 |
| | Basic peak% | 1.2 | 2.4 | 1.6 | 1.3 | 1.3 | 1.3 | 1.3 | 1.2 |

The results show that: at a high temperature of 40 °C, the main peak content of the 2 formulas decreased to a certain extent, and the aggregate content slightly decreased due to degradation of aggregates into part of monomers and fragments; under 25 °C, 2-8 °C, -35 °C, repeated freeze-thaw (-35 °C and room temperature), shaking (200 rpm, 25 °C), and light exposure (25 °C, 5000 lx) conditions, both formulas showed relatively good stability, and under each test condition, the 2 formulas did not significantly differ from each other in stability, indicating that adding 60 mg/mL sucrose and either adding or not adding 0.2 mg/mL polysorbate 80 to the pH 5.2, 10 mM acetic acid-sodium acetate buffer system can improve the freezing and freeze-thaw stability of antibody HER3-29.

**Example 5. Stabilizer Concentration Screening for Antibody HER3-29**

A pH 5.0 or pH 5.4, 10 mM acetic acid-sodium acetate buffer system was selected, 60 mg/mL or 90 mg/mL sucrose was used as a stabilizer, and a total of 4 formulas with an antibody HER3-29 concentration of 20 mg/mL were prepared. The stability of the 4 formulas under high temperature (40 °C), repeated freeze-thaw (-35 °C and room temperature), shaking (200 rpm, 25 °C), and light exposure (25 °C, 5000 lx) conditions was assessed based on the

appearance and the SEC-HPLC purity of the samples.

1) 10 mM acetic acid-sodium acetate, 60 mg/mL sucrose, pH 5.0, 20 mg/mL antibody HER3-29;

2) 10 mM acetic acid-sodium acetate, 90 mg/mL sucrose, pH 5.0, 20 mg/mL antibody HER3-29;

3) 10 mM acetic acid-sodium acetate, 60 mg/mL sucrose, pH 5.4, 20 mg/mL antibody HER3-29;

4) 10 mM acetic acid-sodium acetate, 90 mg/mL sucrose, pH 5.4, 20 mg/mL antibody HER3-29.

Table 7. Results 1 of stabilizer concentration screening for antibody HER3-29

| **No.** | **Appearance** | | | | | | |
|---|---|---|---|---|---|---|---|
| | **T0** | **40 °C** | | **Light exposure** | | **Shaking** | **Freeze-thaw** |
| | | **1W** | **2W** | **5 d** | **10 d** | **48 h** | **3 cycles** |
| 1 | Colorless, clear, free of visible particles | Colorless, clear, free of visible foreign particles | Colorless, clear, free of visible foreign objects | Colorless, clear, free of visible foreign objects | Colorless, clear, free of visible foreign objects | Colorless, clear, free of visible foreign objects | Colorless, clear, free of visible foreign objects |
| 2 | Colorless, clear, free of visible particles | Colorless, clear, free of visible foreign objects | Colorless, clear, free of visible foreign objects | Colorless, clear, free of visible foreign objects | Colorless, clear, free of visible foreign objects | Colorless, clear, free of visible foreign objects | Colorless, clear, free of visible foreign objects |
| 3 | Colorless, clear, free of visible particles | Colorless, clear, free of visible foreign objects | Colorless, clear, free of visible foreign objects | Colorless, clear, free of visible foreign objects | Colorless, clear, free of visible foreign objects | Colorless, clear, free of visible foreign objects | Colorless, clear, free of visible foreign objects |
| 4 | Colorless, clear, free of visible particles | Colorless, clear, free of visible foreign objects | Colorless, clear, free of visible foreign objects | Colorless, clear, free of visible foreign objects | Colorless, clear, free of visible foreign objects | Colorless, clear, free of visible foreign objects | Colorless, clear, free of visible foreign objects |

Table 8. Results 2 of stabilizer concentration screening for antibody HER3-29

| No. | SEC | T0 | 40 °C | | Light exposure | | Shaking | Freeze-thaw |
|---|---|---|---|---|---|---|---|---|
| | | | 1W | 2W | 5 d | 10 d | 48 h | 3 cycles |
| 1 | Monomer% | 94.3 | 95.9 | 95.7 | 94.2 | 94.2 | 94.4 | 94.3 |
| | Aggregate% | 5.6 | 3.1 | 2.8 | 5.6 | 5.7 | 5.5 | 5.6 |
| 2 | Monomer% | 94.3 | 95.8 | 95.8 | 94.2 | 94.2 | 94.4 | 94.3 |
| | Aggregate% | 5.6 | 3.2 | 2.8 | 5.6 | 5.7 | 5.5 | 5.6 |
| 3 | Monomer% | 94.3 | 95.5 | 95.3 | 94.1 | 93.9 | 94.3 | 94.3 |
| | Aggregate% | 5.6 | 3.5 | 3.3 | 5.8 | 5.9 | 5.6 | 5.6 |
| 4 | Monomer% | 94.3 | 95.4 | 95.2 | 94.1 | 94.1 | 94.3 | 94.3 |
| | Aggregate% | 5.6 | 3.7 | 3.3 | 5.7 | 5.8 | 5.6 | 5.6 |

The results show that the appearance of the 4 formulas did not significantly change under the different test conditions. At a high temperature of 40 °C, the aggregate content slightly decreased as aggregates degraded into part of monomers and fragments. Under the other test conditions and at the other time points, neither the monomer content nor the aggregate content changed significantly, indicating that antibody HER3-29 has relatively good stability in the 4

systems. Given that polysorbate 80 may influence the subsequent coupling process, a pH 5.0-5.4 (pH 5.2 ± 0.2), 10 mM acetic acid-sodium acetate system with 60-90 mg/mL sucrose was selected for antibody HER3-29.

**Example 6. Protein Concentration Screening for Antibody HER3-29**

A pH 5.2, 10 mM acetic acid-sodium acetate buffer system was selected, 60 mg/mL sucrose was used as a stabilizer, and a total of 2 formulas with an antibody HER3-29 concentration of 20 mg/mL or 40 mg/mL were prepared. The stability of the 2 formulas under high temperature (40 °C), 25 °C, 2-8 °C, -35 °C, repeated freeze-thaw (-35 °C and room temperature), shaking (200 rpm, 25 °C), and light exposure (25 °C, 5000 lx) conditions was assessed based on the appearance and the SEC-HPLC, iCIEF, and NR-CE purity of the samples.

1) 10 mM acetic acid-sodium acetate, 60 mg/mL sucrose, pH 5.2, 20 mg/mL antibody HER3-29;

2) 10 mM acetic acid-sodium acetate, 60 mg/mL sucrose, pH 5.2, 40 mg/mL antibody HER3-29.

Table 9. Results 1 of protein concentration screening for antibody HER3-29

| No. | Appearance | | | | | | | |
|---|---|---|---|---|---|---|---|---|
| | T0 | 40 °C | 25 °C | 2~8 °C | -35 °C | Freeze-thaw | Shaking | Light exposure |
| | | 4W | 4W | 1M | 1M | 3 cycles | 48 h | 10 d |
| 1 | Colorless, clear, free of visible protein particles | Colorless, clear, free of visible protein particles | Colorless, clear, free of visible protein particles | Colorless, clear, free of visible protein particles | Colorless, clear, a large number of fine bubbles | Colorless, clear, a large number of fine bubbles | Colorless, clear, free of visible protein particles | Colorless, clear, free of visible protein particles |
| 2 | Colorless, clear, free of visible protein particles | Colorless, clear, a small number of protein particles | Colorless, clear, a small number of protein particles | Colorless, clear, free of visible protein particles | Colorless, clear, a large number of fine bubbles | Colorless, clear, a large number of fine bubbles | Colorless, clear, free of visible protein particles | Colorless, clear, a small number of protein particles |

Table 10. Results 2 of protein concentration screening for antibody HER3-29

| No. | SEC | T0 | 40 °C | 25 °C | 2~8 °C | -35 °C | Freeze-thaw | Shaking | Light exposure |
|---|---|---|---|---|---|---|---|---|---|
| | | | 4W | 4W | 1M | 1M | 3 cycles | 48 h | 10 d |
| 1 | Monomer% | 95.7 | 94.9 | 95.7 | 95.6 | 95.6 | 95.8 | 95.8 | 95.5 |
| | Aggregate% | 4.2 | 2.6 | 4.2 | 4.3 | 4.3 | 4.2 | 4.2 | 4.4 |
| 2 | Monomer% | 95.6 | 94.7 | 95.6 | 95.6 | 95.6 | 95.6 | 95.6 | 95.1 |
| | Aggregate% | 4.3 | 2.8 | 4.3 | 4.3 | 4.4 | 4.3 | 4.3 | 4.7 |

Table 11. Results 3 of protein concentration screening for antibody HER3-29

| No. | iCIEF | T0 | 40 °C | 25 °C | 2~8 °C | -35 °C | Freeze-thaw | Shaking | Light exposure |
|---|---|---|---|---|---|---|---|---|---|
| | | | 4W | 4W | 1M | 1M | 3 cycles | 48 h | 10 d |
| 1 | Acidic peak% | 39.7 | 55.2 | 41.9 | 39.5 | 40.3 | 40.3 | 40.1 | 41.7 |
| | Main peak% | 58.6 | 42.2 | 56.5 | 58.7 | 58.2 | 58.1 | 58.2 | 56.5 |
| | Basic peak% | 1.7 | 2.6 | 1.6 | 1.8 | 1.5 | 1.6 | 1.7 | 1.8 |

(continued)

| No. | iCIEF | T0 | 40 °C | 25 °C | 2~8 °C | -35 °C | Freeze-thaw | Shaking | Light exposure |
|---|---|---|---|---|---|---|---|---|---|
| | | | 4W | 4W | 1M | 1M | 3 cycles | 48 h | 10 d |
| 2 | Acidic peak% | 39.2 | 55.8 | 42.1 | 40.2 | 39.8 | 40.4 | 40.5 | 41.7 |
| | Main peak% | 59.0 | 41.5 | 56.2 | 58.4 | 58.8 | 57.9 | 57.7 | 56.4 |
| | Basic peak% | 1.8 | 2.7 | 1.8 | 1.4 | 1.4 | 1.7 | 1.8 | 2.0 |

Table 12. Results 4 of protein concentration screening for antibody HER3-29

| No. | NR-CE | T0 | 40 °C | 25 °C | 2~8 °C | -35 °C | Freeze-thaw | Shaking | Light exposure |
|---|---|---|---|---|---|---|---|---|---|
| | | | 4W | 4W | 1M | 1M | 3 cycles | 48 h | 10d |
| 1 | Main peak% | 97.2 | 93.9 | 96.2 | 97.0 | 96.7 | 97.0 | 97.3 | 97.1 |
| | Fragment% | 2.8 | 6.1 | 3.8 | 3.0 | 3.3 | 3.0 | 2.7 | 2.9 |
| 2 | Main peak% | 97.1 | 93.5 | 96.5 | 96.8 | 96.1 | 96.8 | 97.1 | 97.2 |
| | Fragment% | 2.9 | 6.5 | 3.5 | 3.2 | 3.9 | 3.2 | 2.9 | 2.8 |

The results show that under the different test conditions, the 2 formulas did not significantly differ from each other in SEC, iCIEF, or NR-CE purity, but when the antibody HER3-29 concentration was 40 mg/mL, a small number of protein particles formed in both samples under 40 °C high temperature, 25 °C, and 10-day (10d) light exposure conditions. Therefore, 20 mg/mL was selected as the antibody HER3-29 concentration.

**Example 7. pH Screening for ADC-1**

A 10 mM acetic acid-sodium acetate buffer system was selected, a total of three different pH values, 4.9, 5.2, and 5.5, were designed, 60 mg/mL sucrose was used as a stabilizer, 0.2 mg/mL polysorbate 80 was used as a surfactant, and a total of 3 formulas with an ADC-1 protein concentration of 20.0 mg/mL were prepared. The melting temperature (Tm), the aggregation temperature (Tagg), the particle size (Radius), the SEC-HPLC purity, and the free drug content of the samples were determined, and the stability of the 3 formulas under freeze-thaw (-35 °C and room temperature), shaking (200 rpm, 25 °C), and 40 °C high temperature conditions was assessed.

1) 10 mM acetic acid-sodium acetate, 60 mg/mL sucrose, 0.2 mg/mL polysorbate 80, pH 4.9, 20 mg/mL conjugate ADC-1;
2) 10 mM acetic acid-sodium acetate, 60 mg/mL sucrose, 0.2 mg/mL polysorbate 80, pH 5.2, 20 mg/mL conjugate ADC-1;
3) 10 mM acetic acid-sodium acetate, 60 mg/mL sucrose, 0.2 mg/mL polysorbate 80, pH 5.5, 20 mg/mL conjugate ADC-1.

Table 13. Results 1 of pH screening for ADC-1

| No. | pH | Radius (nm) | %PD | Tm (°C) | Tagg 266 (°C) |
|---|---|---|---|---|---|
| 1 | 4.9 | 2.7 | 8.2 | 63.1 | 60.5 |
| 2 | 5.2 | 3.5 | 6.8 | 63.8 | 63.8 |
| 3 | 5.5 | 4.2 | 7.1 | 63.9 | 62.8 |

Table 14. Results 2 of pH screening for ADC-1

| No. | pH | SEC monomer % | | | | SEC aggregate% | | | |
|---|---|---|---|---|---|---|---|---|---|
| | | Time 0 | Shaking for 3 days | 5 freeze-thaw cycles | 40 °C-2 weeks | Time 0 | Shaking for 3 days | 5 freeze-thaw cycles | 40 °C-2 weeks |
| 1 | 4.9 | 98.0 | 97.9 | 97.8 | 97.8 | 1.8 | 1.9 | 2.0 | 1.6 |
| 2 | 5.2 | 97.9 | 98.0 | 97.7 | 97.6 | 1.9 | 1.8 | 2.1 | 1.9 |
| 3 | 5.5 | 97.9 | 97.8 | 97.7 | 97.3 | 2.0 | 2.0 | 2.1 | 2.2 |

Table 19. Results 3 of pH screening for ADC-1

| No. | Free drug moiety | Time 0 | Shaking | Freeze-thaw | 40 °C |
|---|---|---|---|---|---|
| | | | 3 days | 5 cycles | 2W |
| 1 | 2-B residue (%) | Undetectable | Undetectable | Undetectable | 0.0079 |
| | 9-A residue (%) | Undetectable | Undetectable | Undetectable | 0.0018 |
| | Total(%) | Undetectable | Undetectable | Undetectable | 0.010 |
| 2 | 2-B residue (%) | Undetectable | Undetectable | Undetectable | 0.0056 |
| | 9-A residue (%) | Undetectable | Undetectable | Undetectable | 0.0014 |
| | Total(%) | Undetectable | Undetectable | Undetectable | 0.007 |
| 3 | 2-B residue (%) | Undetectable | Undetectable | Undetectable | 0.0035 |
| | 9-A residue (%) | Undetectable | Undetectable | Undetectable | 0.0026 |
| | Total(%) | Undetectable | Undetectable | Undetectable | 0.006 |
| Note: 2-B is a small-molecule toxin; 9-A is a small-molecule compound linked with a linker; LOD is 0.05 μg/mL, and LOQ is 0.1 μg/mL. %PD refers to the dispersion coefficient, and the smaller the value, the more uniform the particle size distribution; the same applies hereinafter. | | | | | |

**Example 8. Buffer System Screening for ADC-1**

Two buffer systems, 10 mM acetic acid-sodium acetate and 10 mM succinic acid-sodium succinate, pH 5.2, were selected, 60 mg/mL sucrose was used as a stabilizer, 0.2 mg/mL polysorbate 80 was used as a surfactant, and a total of 2 formulas with a HER3 ADC-1 concentration of 20 mg/mL (on a protein basis) were prepared. The stability of the 2 formulas under high temperature (40 °C), 25 °C, and repeated freeze-thaw (-35 °C and room temperature) conditions was assessed based on the appearance, the SEC-HPLC purity, the R-CE purity, the DAR values, and the free drug moiety content of the samples.

1) 10 mM acetic acid-sodium acetate, 60 mg/mL sucrose, 0.2 mg/mL polysorbate 80, pH 5.2, 20 mg/mL conjugate ADC-1;

2) 10 mM succinic acid-sodium succinate, 60 mg/mL sucrose, 0.2 mg/mL polysorbate 80, pH 5.2, 20 mg/mL conjugate ADC-1.

Table 20. Results 1 of buffer system screening for ADC-1

| No. | Appearance | | | | | |
|---|---|---|---|---|---|---|
| | T0 | 25 °C | | 40 °C | | Freeze-thaw |
| | | 1W | 2W | 1W | 2W | 5 cycles |
| 1 | Colorless, clear, free of visible material | Colorless, slightly opalescent, a small number of visible protein particles | Colorless, slightly opalescent, a small number of visible protein particles | Colorless, slightly opalescent, a small number of visible protein particles | Colorless, slightly opalescent, a small number of visible protein particles | Colorless, slightly opalescent, a small number of visible protein particles |
| 2 | Colorless, slightly opalescent, free of visible material | Colorless, slightly opalescent, a small number of visible protein particles | Colorless, slightly opalescent, a large number of visible protein particles | Colorless, slightly opalescent, a small number of visible protein particles | Colorless, slightly opalescent, a large number of visible protein particles | Colorless, slightly opalescent, a small number of visible protein particles |

Table 21. Results 2 of buffer system screening for ADC

| No. | SEC | T0 | 25 °C | | 40 °C | | Freeze-thaw |
|---|---|---|---|---|---|---|---|
| | | | 1W | 2W | 1W | 2W | 5 cycles |
| 1 | Monomer% | 94.2 | 94.1 | 94.0 | 94.1 | 94.3 | 94.0 |
| | Aggregate% | 5.6 | 5.9 | 5.9 | 4.4 | 3.7 | 5.8 |
| 2 | Monomer% | 94.1 | 93.8 | 93.7 | 93.7 | 93.7 | 93.9 |
| | Aggregate% | 5.7 | 6.1 | 6.2 | 4.8 | 4.3 | 6.0 |

Table 22. Results 3 of buffer system screening for ADC

| No. | R-CE | T0 | 25 °C | | 40 °C | | Freeze-thaw |
|---|---|---|---|---|---|---|---|
| | | | 1W | 2W | 1W | 2W | 5 cycles |
| 1 | Main peak% | 98.9 | 99.2 | 99.1 | 98.3 | 98.5 | 99.1 |
| 2 | Main peak% | 99.1 | 99.3 | 99.0 | 98.7 | 97.8 | 99.1 |

Table 23. Results 4 of buffer system screening for ADC

| No. | DAR value | | | | | |
|---|---|---|---|---|---|---|
| | T0 | 25 °C | | 40 °C | | Freeze-thaw |
| | | 1W | 2W | 1W | 2W | 5 cycles |
| 1 | 3.8 | 3.8 | 3.8 | 3.9 | 3.8 | 3.7 |
| 2 | 3.8 | 3.8 | 3.8 | 3.9 | 3.9 | 3.9 |

Table 24. Results 5 of buffer system screening for ADC

| No. | Free drug moiety | T0 | 25 °C | | 40 °C | | Freeze-thaw |
|---|---|---|---|---|---|---|---|
| | | | 1W | 2W | 1W | 2W | 5 cycles |
| 1 | 2-B residue (ppm) | ND | <LOQ | 7.5 | 23.5 | 53.5 | ND |
| | 9-A residue (ppm) | <LOD | ND | ND | ND | ND | ND |

(continued)

| No. | Free drug moiety | T0 | 25 °C | | 40 °C | | Freeze-thaw |
|---|---|---|---|---|---|---|---|
| | | | 1W | 2W | 1W | 2W | 5 cycles |
| 2 | 2-B residue (ppm) | <LOD | <LOQ | 7.5 | 32 | 59.5 | ND |
| | 9-A residue (ppm) | <LOD | ND | ND | ND | ND | ND |
| Note: 2-B is a small-molecule toxin; 9-A is a small-molecule toxin linked with a linker; "ND" stands for "not detectable"; the limit of detection (LOD) is 3 ppm, and the limit of quantitation (LOQ) is 5 ppm. | | | | | | | |

The results show that: in the acetic acid-sodium acetate buffer system and the succinic acid-sodium succinate buffer system, the samples were slightly opalescent with a small number of particles under 25 °C, 40 °C 1 week, and repeated freeze-thaw conditions; in the succinic acid-sodium succinate buffer system, after 2 weeks of standing at 25 °C and 40 °C, the number of protein particles increased; under the same test condition, formula 1 and formula 2 did not significantly differ from each other in DAR value and free drug moiety content, but at 25 °C and 40 °C, formula 1 exhibited slightly higher SEC purity than formula 2; standing at a high temperature of 40 °C for 2 weeks, formula 1 exhibited slightly higher R-CE purity than formula 2, indicating that ADC-1 has better stability in a pH 5.2, 10 mM acetic acid-sodium acetate buffer system containing 60 mg/mL sucrose and 0.2 mg/mL polysorbate 80.

**Example 9. Buffer System Concentration Screening for ADC-1**

Two buffer systems, 10 mM acetic acid-sodium acetate and 20 mM acetic acid-sodium acetate, pH 5.2, were selected, 60 mg/mL sucrose was used as a stabilizer, 0.2 mg/mL polysorbate 80 was used as a surfactant, and a total of 2 formulas with an ADC-1 concentration of 20 mg/mL (on a protein basis) were prepared. The stability of the 2 formulas under shaking (200 rpm, 25 °C), repeated freeze-thaw (-35 °C and room temperature), and high temperature (40 °C) conditions was assessed based on the appearance, the particle size, Tm, Tagg 266, the SEC-HPLC purity, the DAR values, and the free drug moiety content of the samples.

1) 10 mM acetic acid-sodium acetate, 60 mg/mL sucrose, 0.2 mg/mL polysorbate 80, pH 5.2, 20 mg/mL conjugate ADC-1;
2) 20 mM acetic acid-sodium acetate, 60 mg/mL sucrose, 0.2 mg/mL polysorbate 80, pH 5.2, 20 mg/mL conjugate ADC-1.

Table 25. Results 1 of buffer system concentration screening for ADC-1

| No. | Appearance | | | |
|---|---|---|---|---|
| | T0 | Shaking for 3 days | 5 freeze-thaw cycles | 40 °C 2 weeks |
| 1 | Colorless, extremely slightly opalescent, free of visible protein particles | Colorless, extremely slightly opalescent, free of visible protein particles | Colorless, extremely slightly opalescent, free of visible protein particles | Colorless, extremely slightly opalescent, free of visible protein particles |
| 2 | Colorless, extremely slightly opalescent, free of visible protein particles | Colorless, extremely slightly opalescent, free of visible protein particles | Colorless, extremely slightly opalescent, free of visible protein particles | Colorless, extremely slightly opalescent, free of visible protein particles |

Table 26. Results 2 of buffer system concentration screening for ADC

| No. | Radius (nm) | %PD | Tm (°C) | Tagg 266 (°C) |
|---|---|---|---|---|
| 1 | 3.5 | 6.8 | 63.8 | 63.8 |
| 2 | 5.0 | 6.4 | 63.3 | 62.9 |

Table 27. Results 3 of buffer system concentration screening for ADC

| No. | SEC | T0 | Shaking | Freeze-thaw | 40 °C |
|---|---|---|---|---|---|
| | | | 3 days | 5 cycles | 2 weeks |
| 1 | Monomer% | 97.9 | 98.0 | 97.7 | 97.6 |
| | Aggregate% | 1.9 | 1.8 | 2.1 | 1.9 |
| 2 | Monomer% | 97.9 | 97.8 | 97.8 | 97.2 |
| | Aggregate% | 1.9 | 2.0 | 2.1 | 2.3 |

Table 28. Results 4 of buffer system concentration screening for ADC

| No. | DAR value | | | |
|---|---|---|---|---|
| | T0 | Shaking | Freeze-thaw | 40 °C |
| | | 3 days | 5 cycles | 2 weeks |
| 1 | 4.1 | 4.1 | 4.1 | 4.1 |
| 2 | 4.1 | 4.1 | 4.1 | 4.1 |

Table 29. Results 5 of buffer system concentration screening for ADC

| No. | Free drug moiety | Time 0 | Shaking | Freeze-thaw | 40 °C |
|---|---|---|---|---|---|
| | | | 3 days | 5 cycles | 2W |
| 1 | 9-A residue (%) | Undetectable | Undetectable | Undetectable | 0.0014 |
| | 2-B residue (%) | Undetectable | Undetectable | Undetectable | 0.0056 |
| | Total(%) | Undetectable | Undetectable | Undetectable | 0.007 |
| 2 | 9-A residue (%) | Undetectable | Undetectable | Undetectable | 0.0018 |
| | 2-B residue (%) | Undetectable | Undetectable | Undetectable | 0.0059 |
| | Total(%) | Undetectable | Undetectable | Undetectable | 0.008 |
| Note: 2-B is a small-molecule toxin; 9-A is a small-molecule compound linked with a linker; the limit of detection (LOD) is 0.05 μg/mL, and the limit of quantitation (LOQ) is 0.1 μg/mL. | | | | | |

**Example 10. Buffer System Confirmation for ADC-1**

A pH 5.2, 10 mM acetic acid-sodium acetate buffer system was selected, and a sample containing 60 mg/mL sucrose, 0.2 mg/mL polysorbate 80, and 20 mg/mL ADC-1 was prepared. The stability of the formula under high temperature (40 °C), 25 °C, 2-8 °C, - 35 °C, repeated freeze-thaw (-35 °C and room temperature), shaking (200 rpm, 25 °C), and light exposure (25 °C, 5000 lx) conditions was assessed based on the SEC-HPLC purity, the R-CE purity, the DAR value, the free drug moiety content, and the binding activity of the sample.

10 mM acetic acid-sodium acetate, 60 mg/mL sucrose, 0.2 mg/mL polysorbate 80, pH 5.2, 20 mg/mL conjugate ADC-1.

Table 30. Results 1 of buffer system confirmation for ADC-1

| No. | Appearance | | | |
|---|---|---|---|---|
| | T0 | Shaking for 3 days | 5 freeze-thaw cycles | 40 °C 2 weeks |
| 1 | Colorless, extremely slightly opalescent, free of visible protein particles | Colorless, extremely slightly opalescent, free of visible protein particles | Colorless, extremely slightly opalescent, free of visible protein particles | Colorless, extremely slightly opalescent, free of visible protein particles |

Table 31. Results 2 of buffer system confirmation for ADC-1

| No. | Radius (nm) | %PD | Tm (°C) | Tagg 266 (°C) |
|---|---|---|---|---|
| 1 | 3.5 | 6.8 | 63.8 | 63.8 |

Table 32. Results 3 of buffer system confirmation for ADC-1

| No. | SEC | T0 | 40 °C | 25 °C | 2~8 °C | -35 °C | Freeze-thaw | Shaking | Light exposure |
|---|---|---|---|---|---|---|---|---|---|
| | | | 4W | 4W | 3M | 3M | 5 cycles | 24 h | 5d |
| 1 | Monomer% | 96.0 | 95.8 | 96.2 | 96.4 | 96.6 | 96.1 | 96.1 | 91.8 |
| | Aggregate% | 3.9 | 3.8 | 3.8 | 3.5 | 3.3 | 3.8 | 3.7 | 6.5 |

Table 33. Results 4 of buffer system confirmation for ADC-1

| No. | R-CE | T0 | 40 °C | 25 °C | 2~8 °C | -35 °C | Freeze-thaw | Shaking | Light exposure |
|---|---|---|---|---|---|---|---|---|---|
| | | | 4W | 4W | 3M | 3M | 5 cycles | 24 h | 5 d |
| 1 | Main peak% | 99.4 | 98.7 | 98.7 | 99.4 | 99.4 | 98.9 | 98.5 | 91.6 |

Table 34. Results 5 of buffer system confirmation for ADC-1

| **No.** | **DAR value** | | | | | | | |
|---|---|---|---|---|---|---|---|---|
| | **T0** | **40 °C** | **25 °C** | **2~8 °C** | **-35 °C** | **Freeze-thaw** | **Shaking** | **Light exposure** |
| | | **4W** | **4W** | **3M** | **3M** | **5 cycles** | **24 h** | **5 d** |
| 1 | 4.0 | 3.8 | 3.9 | 3.9 | 4.0 | 4.0 | 4.0 | 4.3 |

Table 35. Results 6 of buffer system confirmation for ADC-1

| No. | Free drug moiety | T0 | 40 °C | 25 °C | 2~8 °C | -35 °C | Freeze-thaw | Shaking | Light exposure |
|---|---|---|---|---|---|---|---|---|---|
| | | | 4W | 4W | 3M | 3M | 5 cycles | 24 h | 5 d |
| 1 | 2-B residue (ppm) | <LOD | 91 | 10 | <5.3 | <LOD | <LOD | <LOQ | <LOQ |
| | 9-A residue (ppm) | 43.6 | <LOQ | ND | <LOD | 91.8 | 51.5 | 18.6 | <LOQ |
| Note: ND stands for not detectable; the limit of detection (LOD) is 3 ppm, and the limit of quantitation (LOQ) is 5 ppm. | | | | | | | | | |

The results show that: the repeated freeze-thaw, -35 °C storage, and shaking conditions did not significantly influence the protein monomer and aggregate contents, the R-CE purity, the DAR value, and the free drug moiety content; standing under light exposure conditions for 5 days, the sample exhibited a relatively significant decrease in monomer content, a certain increase in DAR value, and a decrease in R-CE purity; therefore, the sample should be protected from light during storage and transportation.

**Example 11. ADC-1 Concentration Screening**

A pH 5.2, 10 mM acetic acid-sodium acetate system was selected, 60 mg/mL sucrose was used as a stabilizer, 0.2 mg/mL polysorbate 80 was used as a surfactant, and a total of 2 formulas with ADC-1 concentrations of 20 mg/mL and 40 mg/mL (on a protein basis) were prepared. The stability of the 2 formulas under shaking (200 rpm, 25 °C), repeated freeze-thaw (-35 °C and room temperature), and high temperature (40 °C) conditions was assessed based on the appearance, the particle size, Tm, Tagg 266, the SEC-HPLC purity, the DAR values, and the free drug moiety content of the samples.

1) 10 mM acetic acid-sodium acetate, 60 mg/mL sucrose, 0.2 mg/mL polysorbate 80, pH 5.2, 20 mg/mL conjugate ADC-1;

2) 10 mM acetic acid-sodium acetate, 60 mg/mL sucrose, 0.2 mg/mL polysorbate 80, pH 5.2, 40 mg/mL conjugate

ADC-1.

Table 36. Results 1 of ADC-1 concentration screening

| No. | Appearance | | | |
|---|---|---|---|---|
| | T0 | Shaking for 3 days | 5 freeze-thaw cycles | 40°C 2W |
| 1 | Colorless, extremely slightly opalescent, free of visible protein particles | Colorless, extremely slightly opalescent, free of visible protein particles | Colorless, extremely slightly opalescent, free of visible protein particles | Colorless, extremely slightly opalescent, free of visible protein particles |
| 2 | Colorless, extremely slightly opalescent, free of visible protein particles | Colorless, extremely slightly opalescent, free of visible protein particles | Colorless, extremely slightly opalescent, free of visible protein particles | Colorless, extremely slightly opalescent, free of visible protein particles |

Table 37. Results 2 of ADC-1 concentration screening

| No. | Radius (nm) | %PD | Tm (°C) | Tagg 266 (°C) |
|---|---|---|---|---|
| 1 | 3.5 | 6.8 | 63.8 | 63.8 |
| 2 | 2.8 | 9.0 | 63.3 | 59.1 |

Table 38. Results 3 of ADC-1 concentration screening

| No. | SEC | T0 | Shaking | Freeze-thaw | 40 °C |
|---|---|---|---|---|---|
| | | | 3 days | 5 cycles | 2W |
| 1 | Monomer% | 97.9 | 98.0 | 97.7 | 97.6 |
| | Aggregate% | 1.9 | 1.8 | 2.1 | 1.9 |
| 2 | Monomer% | 97.8 | 97.8 | 97.7 | 97.2 |
| | Aggregate% | 2.0 | 2.1 | 2.2 | 2.3 |

Table 39. Results 4 of ADC-1 concentration screening

| No. | DAR value | | | |
|---|---|---|---|---|
| | T0 | Shaking | Freeze-thaw | 40 °C |
| | | 3 days | 5 cycles | 2W |
| 1 | 4.1 | 4.1 | 4.1 | 4.1 |
| 2 | 4.2 | 4.2 | 4.1 | 4.1 |

Table 40. Results 5 of ADC-1 concentration screening

| No. | Free drug moiety | Time 0 | Shaking | Freeze-thaw | 40 °C |
|---|---|---|---|---|---|
| | | | 3 days | 5 cycles | 2W |
| 1 | 9-A residue (%) | Undetectable | Undetectable | Undetectable | 0.0014 |
| | 2-B residue (%) | Undetectable | Undetectable | Undetectable | 0.0056 |
| | Total(%) | Undetectable | Undetectable | Undetectable | 0.007 |

(continued)

| No. | Free drug moiety | Time 0 | Shaking | Freeze-thaw | 40 °C |
|---|---|---|---|---|---|
| | | | 3 days | 5 cycles | 2W |
| 2 | 9-A residue (%) | Undetectable | Undetectable | Undetectable | 0.0018 |
| | 2-B residue (%) | Undetectable | <0.0005 | Undetectable | 0.0055 |
| | Total(%) | Undetectable | <0.001 | Undetectable | 0.007 |
| Note: 2-B is a small-molecule toxin; 9-A is a small-molecule compound linked with a linker; the limit of detection (LOD) is 0.05 μg/mL, and the limit of quantitation (LOQ) is 0.1 μg/mL. | | | | | |

**Example 12. Stabilizer Concentration and Surfactant Concentration Screening for ADC-1**

ApH 5.2, 10 mM acetic acid-sodium acetate buffer system was selected, and a total of 2 formula samples containing 60 mg/mL or 120 mg/mL sucrose, 0.2 mg/mL or 0.4 mg/mL polysorbate 80, and 20 mg/mL ADC-1 were prepared. The samples were filtered through a 0.22 μm filter and added to 20-mL vials at 5.3 mL. The vials were half plugged, subjected to a lyophilization process using the parameters shown in the table below, fully plugged, and capped. The appearance, water content, reconstitution time, SEC purity, R-CE purity, DAR value, and free drug moiety content were determined, and the difference in product quality between a formula before lyophilization and the same formula after lyophilization was determined to assess the influence of the lyophilization process on product quality; meanwhile, the different formulas were compared in terms of quality to screen for a stabilizer concentration and a surfactant concentration.

1) 10 mM acetic acid-sodium acetate, 60 mg/mL sucrose, 0.2 mg/mL polysorbate 80, pH 5.2, 20 mg/mL ADC-1;
2) 10 mM acetic acid-sodium acetate, 120 mg/mL sucrose, 0.4 mg/mL polysorbate 80, pH 5.2, 20 mg/mL ADC-1.

Table 41. Lyophilization process parameters for ADC-1

| Lyophilization program | Set temperature (°C) | Set time (min) | Holding time (min) | Vacuum degree (Pa) |
|---|---|---|---|---|
| Feeding in | 5 | N/A | N/A | N/A |
| Pre-freezing | -5 | 10 | 60 | N/A |
| Pre-freezing | -45 | 50 | 120 | N/A |
| Primary drying | -20 | 60 | 2400 | 10 |
| Secondary drying | 25 | 60 | 260 | 1 |

Table 42. Results 1 of stabilizer concentration and surfactant concentration screening for ADC-1

| Sample name | State | Appearance | Water% | Reconstitution time |
|---|---|---|---|---|
| DS-1 | Liquid | Colorless, clear, free of visible protein particles | NA | NA |
| DP-1 | Solid | Off-white block | 1.0 | 2 min and 18 s |
| DS-2 | Liquid | Colorless, clear, free of visible protein particles | NA | NA |
| DP-2 | Solid | Off-white block | 1.7 | 2 min and 55 s |
| Note: For formula 1, the sample before lyophilization is designated DS-1, and the sample after lyophilization is designated DP-1; for formula 2, the sample before lyophilization is designated DS-2, and the sample after lyophilization is designated DP-2. | | | | |

Table 43. Results 2 of stabilizer concentration and surfactant concentration screening for ADC-1

| Sample name | DAR value | R-CE | SEC | | Free drug moiety | |
|---|---|---|---|---|---|---|
| | | Main peak% | Monomer | Aggregate% | 2-B residue (ppm) | 9-A residue (ppm) |
| DS-1 | 4.0 | 99.4 | 96.0 | 3.9 | <3 | 43.6 |

(continued)

| Sample name | DAR value | R-CE | SEC | | Free drug moiety | |
|---|---|---|---|---|---|---|
| | | Main peak% | Monomer | Aggregate% | 2-B residue (ppm) | 9-A residue (ppm) |
| DP-1 | 4.0 | 99.4 | 96.1 | 3.8 | <3 | 6 |
| DS-2 | 4.0 | 99.4 | 96.5 | 3.5 | <3 | 77.3 |
| DP-2 | 4.0 | 99.4 | 96.2 | 3.6 | <3 | 12 |
| Note: For formula 1, the sample before lyophilization is designated DS-1, and the sample after lyophilization is designated DP-1; for formula 2, the sample before lyophilization is designated DS-2, and the sample after lyophilization is designated DP-2. | | | | | | |

The results show that: for the 2 formulas, both the appearances of the samples before lyophilization and the appearances of the samples after lyophilization were acceptable; compared to the sample before lyophilization, the sample of the same formula after lyophilization had a lower free drug moiety content, and there was no significant change in monomer content, aggregate content, DAR value, and R-CE purity, indicating that this lyophilization process did not significantly influence product quality; the freeze-dried products of the 2 formulas did not significantly differ from each other in each test item. Therefore, the sucrose concentration was 60-120 mg/mL, and the polysorbate 80 concentration was 0.2-0.4 mg/mL.

**Example 13. Stabilizer Concentration and Surfactant Concentration Confirmation 1 for ADC-1**

A pH 5.2, 10 mM acetic acid-sodium acetate buffer system was selected, and a formula sample containing 60 mg/mL sucrose, 0.2 mg/mL polysorbate 80, and 20 mg/mL ADC-1 was prepared. The sample was filtered through a 0.22 μm filter and added to a 20-mL vial at 5.3 mL. The vial was half plugged, subjected to a lyophilization process using the parameters shown in the table below, fully plugged, and capped. The appearance, water content, reconstitution time, SEC purity, R-CE purity, DAR value, and free drug moiety content were determined to confirm the stabilizer concentration and the surfactant concentration.

Table 44. Stabilizer concentration and surfactant concentration confirmation 1 lyophilization process parameters for ADC-1

| Lyophilization program | Set temperature (°C) | Set time (min) | Holding time (min) | Vacuum degree (Pa) |
|---|---|---|---|---|
| Sample loading | 5 | N/A | N/A | N/A |
| Pre-freezing | -5 | 10 | 60 | N/A |
| Pre-freezing | -45 | 40 | 180 | N/A |
| Primary drying | -20 | 120 | 2400 | 10 |
| Secondary drying | 25 | 60 | 480 | 5 |

Table 45. Results 1 of stabilizer concentration and surfactant concentration confirmation 1 for ADC-1

| Sample name | State | Appearance | Water% | Reconstitution time |
|---|---|---|---|---|
| DS-1 | Liquid | Colorless, clear, free of visible protein particles | N/A | N/A |
| DP-1 | Solid | Off-white block | 0.6 | 3 min and 47 s |
| Note: For formula 1, the sample before lyophilization is designated DS-1, and the sample after lyophilization is designated DP-1. | | | | |

Table 46. Results 2 of stabilizer concentration and surfactant concentration confirmation 1 for ADC-1

| Sample name | DAR value | R-CE | SEC | | Free drug moiety | |
|---|---|---|---|---|---|---|
| | | Main peak% | Monomer | Aggregate% | 2-B residue (ppm) | 9-A residue (ppm) |
| DS-1 | 3.6 | 99.4 | 95.0 | 4.9 | <2.5 | <2.5 |

(continued)

| Sample name | DAR value | R-CE | SEC | | Free drug moiety | |
|---|---|---|---|---|---|---|
| | | Main peak% | Monomer | Aggregate% | 2-B residue (ppm) | 9-A residue (ppm) |
| DP-1 | 3.6 | 99.4 | 94.9 | 5.0 | <2.5 | <2.5 |
| Note: For formula 1, the sample before lyophilization is designated DS-1, and the sample after lyophilization is designated DP-1. | | | | | | |

The results show that after the pre-freezing time was extended from 120 min to 180 min, the secondary drying time was extended from 260 min to 480 min, and the vacuum degree of secondary drying was adjusted from 1 pa to 5 pa, the sample after lyophilization did not significantly differ from the sample before lyophilization in free drug moiety content, monomer content, aggregate content, DAR value, and R-CE purity, indicating that this lyophilization process did not significantly influence product quality, and that the formula has relatively good stability.

**Example 14. Stabilizer Concentration and Surfactant Concentration Confirmation 2 for ADC-1**

A pH 5.2, 10 mM acetic acid-sodium acetate buffer system was selected, and a formula sample containing 120 mg/mL sucrose, 0.4 mg/mL polysorbate 80, and 20 mg/mL ADC-1 was prepared. The sample was filtered through a 0.22 μm filter and added to a 20-mL vial at 5.3 mL. The vial was half plugged, subjected to a lyophilization process using the parameters shown in the table below, fully plugged, and capped. The appearance, water content, reconstitution time, SEC purity, R-CE purity, DAR value, and free drug moiety content were determined to confirm the stabilizer concentration and the surfactant concentration.

Table 47. Stabilizer concentration and surfactant concentration confirmation 2 lyophilization process parameters for ADC-1

| Lyophilization program | Set temperature (°C) | Set time (min) | Holding time (min) | Vacuum degree (Pa) |
|---|---|---|---|---|
| Sample loading | 5 | N/A | N/A | N/A |
| Pre-freezing | -5 | 10 | 60 | N/A |
| Pre-freezing | -45 | 50 | 120 | N/A |
| Primary drying | -15 | 120 | 2100 | 10 |
| Secondary drying | 25 | 60 | 480 | 1 |

Table 48. Results 1 of stabilizer concentration and surfactant concentration confirmation 2 for ADC-1

| Sample name | State | Appearance | Water% | Reconstitution time |
|---|---|---|---|---|
| DS-1 | Liquid | Colorless, clear, free of visible protein particles | N/A | N/A |
| DP-1 | Solid | Off-white block | 1.0 | 3 min and 13 s |
| Note: For formula 1, the sample before lyophilization is designated DS-1, and the sample after lyophilization is designated DP-1. | | | | |

Table 49. Results 2 of stabilizer concentration and surfactant concentration confirmation 2 for ADC-1

| Sample name | DAR value | R-CE | SEC | | Free drug moiety | |
|---|---|---|---|---|---|---|
| | | Main peak% | Monomer | Aggregate% | 2-B residue (ppm) | 9-A residue (ppm) |
| DS-1 | 4.0 | 99.4 | 96.5 | 3.5 | <3 | 77.3 |
| DP-1 | 4.0 | 99.4 | 96.7 | 3.3 | <3 | <5 |
| Note: For formula 1, the sample before lyophilization is designated DS-1, and the sample after lyophilization is designated DP-1. | | | | | | |

The results show that after the primary drying temperature was increased from -20 °C to -15 °C, the primary

drying time was shortened from 2400 min to 2100 min, and the secondary drying time was extended from 240 min to 480 min, the sample after lyophilization did not significantly differ from the sample before lyophilization in monomer content, aggregate content, DAR value, and R-CE purity except that the sample after lyophilization had a lower free drug moiety content, indicating that this lyophilization process did not significantly influence product quality, and that the formula has relatively good stability.

**Example 15. Alternative Anti-HER3 Antibody Formulation Formulas**

The present disclosure provides formulas of "18-22 mg/mL anti-HER3 antibody HER3-29 drug, 60-90 mg/mL sucrose, 10 mM acetic acid-sodium acetate, pH 5.0-5.4", including but not limited to:

1) 20 mg/mL anti-HER3 antibody, 60 mg/mL sucrose, 10 mM acetic acid-sodium acetate, pH 5.0;
2) 20 mg/mL anti-HER3 antibody, 60 mg/mL sucrose, 10 mM acetic acid-sodium acetate, pH 5.2;
3) 20 mg/mL anti-HER3 antibody, 60 mg/mL sucrose, 10 mM acetic acid-sodium acetate, pH 5.4;
4) 20 mg/mL anti-HER3 antibody, 90 mg/mL sucrose, 10 mM acetic acid-sodium acetate, pH 5.0;
5) 20 mg/mL anti-HER3 antibody, 90 mg/mL sucrose, 10 mM acetic acid-sodium acetate, pH 5.2;
6) 20 mg/mL anti-HER3 antibody, 90 mg/mL sucrose, 10 mM acetic acid-sodium acetate, pH 5.4.

**Example 16. Alternative Anti-HER3 Antibody-Drug Conjugate Formulation Formulas**

The present disclosure provides formulation formulas of "18-22 mg/mL anti-HER3 antibody-drug conjugate drug, 60-120 mg/mL sucrose, 0.2-0.4 mg/mL polysorbate 80, 10 mM acetic acid-sodium acetate, pH 5.0-5.4", including but not limited to:

1) 20 mg/mL ADC-1, 60 mg/mL sucrose, 0.2 mg/mL polysorbate 80, 10 mM acetic acid-sodium acetate, pH 5.0;
2) 20 mg/mL ADC-1, 60 mg/mL sucrose, 0.2 mg/mL polysorbate 80, 10 mM acetic acid-sodium acetate, pH 5.2;
3) 20 mg/mL ADC-1, 60 mg/mL sucrose, 0.2 mg/mL polysorbate 80, 10 mM acetic acid-sodium acetate, pH 5.4;
4) 20 mg/mL ADC-1, 120 mg/mL sucrose, 0.2 mg/mL polysorbate 80, 10 mM acetic acid-sodium acetate, pH 5.0;
5) 20 mg/mL ADC-1, 120 mg/mL sucrose, 0.2 mg/mL polysorbate 80, 10 mM acetic acid-sodium acetate, pH 5.2;
6) 20 mg/mL ADC-1, 120 mg/mL sucrose, 0.2 mg/mL polysorbate 80, 10 mM acetic acid-sodium acetate, pH 5.4;
7) 20 mg/mL ADC-1, 60 mg/mL sucrose, 0.4 mg/mL polysorbate 80, 10 mM acetic acid-sodium acetate, pH 5.0;
8) 20 mg/mL ADC-1, 60 mg/mL sucrose, 0.4 mg/mL polysorbate 80, 10 mM acetic acid-sodium acetate, pH 5.2;
9) 20 mg/mL ADC-1, 60 mg/mL sucrose, 0.4 mg/mL polysorbate 80, 10 mM acetic acid-sodium acetate, pH 5.4;
10) 20 mg/mL ADC-1, 120 mg/mL sucrose, 0.4 mg/mL polysorbate 80, 10 mM acetic acid-sodium acetate, pH 5.0;
11) 20 mg/mL ADC-1, 120 mg/mL sucrose, 0.4 mg/mL polysorbate 80, 10 mM acetic acid-sodium acetate, pH 5.2;
12) 20 mg/mL ADC-1, 120 mg/mL sucrose, 0.4 mg/mL polysorbate 80, 10 mM acetic acid-sodium acetate, pH 5.4.

## Claims

1. A pharmaceutical composition, comprising an anti-HER3 antibody-drug conjugate, wherein the anti-HER3 antibody-drug conjugate has a structure represented by the following formula:

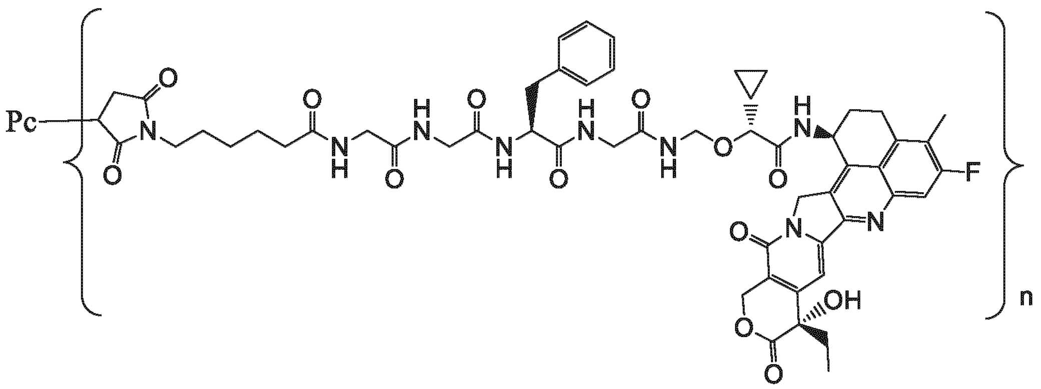

,

wherein:

n is 1 to 8;
Pc is an anti-HER3 antibody comprising a heavy chain variable region and a light chain variable region, wherein:

the heavy chain variable region comprises a HCDR1, a HCDR2, and a HCDR3 set forth in SEQ ID NO: 1, SEQ ID NO: 2, and SEQ ID NO: 3, respectively, and the light chain variable region comprises a LCDR1, a LCDR2, and a LCDR3 set forth in SEQ ID NO: 4, SEQ ID NO: 5, and SEQ ID NO: 6, respectively;
the pharmaceutical composition further comprises an acetate buffer, preferably an acetic acid-sodium acetate buffer, and the pharmaceutical composition has a pH of 4.9 to 5.5, preferably a pH of about 5.2.

**2.** The pharmaceutical composition according to claim 1, wherein the anti-HER3 antibody comprises:

a heavy chain variable region set forth in SEQ ID NO: 7 and a light chain variable region set forth in SEQ ID NO: 8;
preferably, the anti-HER3 antibody comprises:
a heavy chain set forth in SEQ ID NO: 9 and a light chain set forth in SEQ ID NO: 10.

**3.** The pharmaceutical composition according to claim 1 or 2, wherein n is 3 to 5; preferably, n is about 4.

**4.** The pharmaceutical composition according to any one of claims 1 to 3, wherein the pharmaceutical composition further comprises a surfactant; the surfactant is preferably a polysorbate, more preferably polysorbate 80 or polysorbate 20, and most preferably polysorbate 80.

**5.** The pharmaceutical composition according to claim 4, wherein the surfactant is at a concentration of 0.05 mg/mL to 0.6 mg/mL, preferably 0.1 mg/mL to 0.4 mg/mL, and more preferably about 0.2 mg/mL.

**6.** The pharmaceutical composition according to any one of claims 1 to 5, wherein the composition further comprises a sugar; preferably, the sugar is selected from the group consisting of sucrose and $\alpha,\alpha$-trehalose dihydrate; more preferably, the sugar is sucrose.

**7.** The pharmaceutical composition according to claim 6, wherein the sugar is at a concentration of 10 mg/mL to 150 mg/mL, preferably 40 mg/mL to 120 mg/mL, more preferably 40 mg/mL to 80 mg/mL, and most preferably about 60 mg/mL.

**8.** The pharmaceutical composition according to any one of claims 1 to 7, wherein the buffer is at a concentration of 1 mM to 50 mM, preferably 5 mM to 15 mM, and more preferably about 10 mM.

**9.** The pharmaceutical composition according to any one of claims 1 to 8, wherein the antibody-drug conjugate is at a concentration of 1 mg/mL to 40 mg/mL, preferably 10 mg/mL to 30 mg/mL, more preferably 15 mg/mL to 25 mg/mL, and most preferably about 20 mg/mL.

**10.** The pharmaceutical composition according to any one of claims 1 to 9, wherein the pharmaceutical composition comprises the following components:

(a) 1 mg/mL to 40 mg/mL said anti-HER3 antibody-drug conjugate, (b) 0.05 mg/mL to 0.6 mg/mL polysorbate, (c) 10 mg/mL to 150 mg/mL sugar, and (d) 1 mM to 50 mM acetate buffer; the pharmaceutical composition has a pH of 4.9-5.5;
preferably, the pharmaceutical composition comprises the following components:

(a) 10 mg/mL to 30 mg/mL said anti-HER3 antibody-drug conjugate, (b) 0.1 mg/mL to 0.4 mg/mL polysorbate, (c) 40 mg/mL to 120 mg/mL sugar, and (d) 5 mM to 15 mM acetate buffer; the pharmaceutical composition has a pH of 5.0-5.5;
more preferably, the pharmaceutical composition comprises the following components: (a) 15 mg/mL to 25 mg/mL said anti-HER3 antibody-drug conjugate, (b) 0.1 mg/mL to 0.4 mg/mL polysorbate 80, (c) 40 mg/mL to 80 mg/mL sucrose, and (d) 5 mM to 15 mM acetic acid-sodium acetate buffer; the pharmaceutical composition has a pH of 5.1-5.3; most preferably, the pharmaceutical composition comprises the following components: (a) about 20 mg/mL said anti-HER3 antibody-drug conjugate, (b) about 0.2 mg/mL polysorbate 80, (c) about 60 mg/mL sucrose, and (d) about 10 mM acetic acid-sodium acetate buffer; the pharmaceutical composition has a pH of about 5.2.

**11.** A freeze-dried formulation comprising an anti-HER3 antibody-drug conjugate, wherein the formulation is capable of being reconstituted to form the pharmaceutical composition according to any one of claims 1 to 10.

**12.** A method for preparing a freeze-dried formulation comprising an anti-HER3 antibody-drug conjugate, comprising a step of lyophilizing the pharmaceutical composition according to any one of claims 1 to 10.

**13.** A method for treating a disease, comprising administering to a patient an effective amount of the pharmaceutical composition according to any one of claims 1 to 10 or the freeze-dried formulation according to claim 11, wherein: preferably, the disease is a tumor or cancer; more preferably, the disease is selected from the group consisting of breast cancer, non-small cell lung cancer, gastric cancer, ovarian cancer, prostate cancer, bladder cancer, colorectal cancer, head and neck squamous cell carcinoma, and melanoma.

## INTERNATIONAL SEARCH REPORT

International application No.
**PCT/CN2023/134686**

**A. CLASSIFICATION OF SUBJECT MATTER**

A61K47/68(2017.01)i; A61K39/395(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: A61K, A61P

Documentation searched other than minimum documentation to the extent that such documents are included in the fields searched

Electronic data base consulted during the international search (name of data base and, where practicable, search terms used)

CNABS; DWPI; VEN; CNTXT; WOTXT; EPTXT; USTXT; CNKI; 万方数据知识服务平台, WANFANG DATA KNOWLEDGE SERVICE PLATFORM; STN; Web of Science; Elsevier Science Direct; PubMed; 百度学术, Baidu Scholar: 结构式检索, structural formula search, HER3, 抗体偶联物, antibody drug conjugate, ADC

**C. DOCUMENTS CONSIDERED TO BE RELEVANT**

| Category* | Citation of document, with indication, where appropriate, of the relevant passages | Relevant to claim No. |
|---|---|---|
| X | WO 2022078425 A1 (JIANGSU HENGRUI MEDICINE CO., LTD.; SHANGHAI HENGRUI PHARMACEUTICAL CO., LTD.;) 21 April 2022 (2022-04-21) description, pages 2, 3 and 10 | 1-13 |
| X | WO 2020063676 A1 (JIANGSU HENGRUI MEDICINE CO., LTD.; SHANGHAI HENGRUI PHARMACEUTICAL CO., LTD.;) 02 April 2020 (2020-04-02) description, page 12, tables, and page 33, and claims 25 and 26 | 1-13 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **20 February 2024** | **03 March 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/134686**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2023/134686**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **13**
because they relate to subject matter not required to be searched by this Authority, namely:

Claim 13 relates to a method for treatment of a disease, which falls within subject matter for which no search is performed (PCT Rule 39.1(iv)), but a search is still carried out in the present report regarding a corresponding pharmaceutical use thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/134686**

| Patent document cited in search report | Publication date (day/month/year) | Patent family member(s) | Publication date (day/month/year) |
|---|---|---|---|
| WO 2022078425 A1 | 21 April 2022 | CA 3196940 A1 | 21 April 2022 |
| | | JP 2023545925 A | 01 November 2023 |
| | | AU 2021360625 A1 | 01 June 2023 |
| | | KR 20230086702 A | 15 June 2023 |
| | | TW 202304983 A | 01 February 2023 |
| | | EP 4230653 A1 | 23 August 2023 |
| WO 2020063676 A1 | 02 April 2020 | ZA 202102544 B | 25 October 2023 |
| | | CA 3114137 A1 | 02 April 2020 |
| | | BR 112021004656 A2 | 01 June 2021 |
| | | JP 2022511351 A | 31 January 2022 |
| | | EP 3858386 A1 | 04 August 2021 |
| | | EP 3858386 A4 | 12 October 2022 |
| | | KR 20210066833 A | 07 June 2021 |
| | | MX 2021003382 A | 27 May 2021 |
| | | US 2021353764 A1 | 18 November 2021 |
| | | AU 2019349207 A1 | 08 April 2021 |
| | | TW 202027795 A | 01 August 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20050238649 A1
- US 5208020 A
- WO 2022078425 A1
- WO 2020063676 A1


### Non-patent literature cited in the description


- **CHARI et al.** *Cancer Research*, 1992, vol. 52, 127-131
- *J. Biol. Chem.*, 1968, vol. 243, 3558
- Antibodies: A Laboratory Manual and Molecular Cloning: A Laboratory Manual. Cold Spring Harbor Laboratory